# EUROPÄISCHE PATENTSCHRIFT

(11) **EP 1 819 708 B1**
(45) Veröffentlichungstag und Bekanntmachung des Hinweises auf die Patenterteilung: **11.01.2012**
(21) Anmeldenummer: 05849039.2
(22) Anmeldetag: 25.11.2005
(51) Int. Cl.: C07D 475/08, A61K 31/519, A61P 35/00

(54) **SUBSTITUIERTE PTERIDINE ZUR BEHANDLUNG VON ENTZÜNDLICHEN ERKRANKUNGEN**
SUBSTITUTED PTERIDINES FOR TREATING INFLAMMATORY DISEASES
PTERIDINES SUBSTITUEES DESTINEES AU TRAITEMENT DES MALADIES INFLAMMATOIRES

(30) Priorität: 29.11.2004 DE 102004057594
(43) Veröffentlichungstag der Anmeldung: 22.08.2007
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: DOLLINGER, Horst, 88433 Schemmerhofen (DE); MARTYRES, Domnic, 88400 Biberach (DE); MACK, Juergen, 88400 Biberach (DE); NICKOLAUS, Peter, 88447 Warthausen (DE); JUNG, Birgit, 88471 Laupheim (DE)
(74) Vertreter: Hammann, Heinz
(86) Internationale Anmeldenummer: PCT/EP2005/056245
(87) Internationale Veröffentlichungsnummer: WO 2006/058868

(56) Entgegenhaltungen:
- EP-A- 0 185 259
- WO-A-03/062240
- DE-A1- 3 540 952
- MERZ K-H ET AL: "Synthesis of 7-Benzylamino-6-chloro-2-piperazino-4-pyrr olidinopterid ine and Novel Derivatives Free of Positional Isomers. Potent Inhibitors of cAMP-Specific Phosphodiesterase and of Malignant Tumor Cell Growth" JOURNAL OF MEDICINAL CHEMISTRY, AMERICAN CHEMICAL SOCIETY. WASHINGTON, US, Bd. 41, Nr. 24, 1998, Seiten 4733-4743, XP002239611 ISSN: 0022-2623 in der Anmeldung erwähnt

## Beschreibung

Die Erfindung betrifft neue Pteridine, die geeignet sind zur Behandlung von
- Atemwegs- oder gastrointestinalen Beschwerden oder Erkrankungen,
- entzündlichen Erkrankungen der Gelenke, der Haut oder der Augen,
- Erkrankungen des periphären oder zentralen Nervensystems oder
- Krebserkrankungen,
sowie pharmazeutische Zusammensetzungen die diese Verbindungen beinhalten.

### STAND DER TECHNIK

Pteridine sind als Wirkstoffe mit antiproliferativer Wirkung aus dem Stand der Technik bekannt. Merz et al. beschreiben im Journal of Medicinal Chemistry 1998, 41, 4733-4743 die Herstellung von 7-Benzylamino-6-chlor-2-piperazino-4- pyrrolidinopteridin und Derivaten davon, welche frei von Stellungsisomeren sind. Es wurde gezeigt, dass die hergestellten Verbindungen das Wachstum von Tumorzellen hemmen können. In der DE 3540952 werden 2-Piperazino-pteridine beschrieben, die in der 6-Stellung mit einem Halogenatom, ausgewählt aus einem Fluor-, Chlor- oder Bromatom, substituiert sind. Es wurde gezeigt, dass diese Verbindungen die Aktivität von Tumorzellen und von Humanthrombozyten in vitro hemmen konnten. Die DE 3323932 offenbart 2-Piperazino-pteridine, die in der 4-Stellung eine Dialkylamino-, Piperidino-, Morpholino-, Thiomorpholino- oder 1-Oxidothiomorpholinogruppe tragen. Es wurde gezeigt, dass diese Verbindungen die Aktivität von Tumorzellen und von Humanthrombozyten in vitro hemmen konnten. In der DE 3445298 werden Pteridine mit einer großen Anzahl an unterschiedlichen Substituenten in der 2-, 4-, 6- und 7-Stellung beschrieben, wobei sich Verbindungen mit einer 2-Piperazinogruppe am Pteridingerüst als Hemmstoffe für das Tumorwachstum eignen sowie antithrombotische und metastasenhemmende Eigenschaften aufweisen. In der US 2,940,972 werden tri- und tetrasubstituierte Pteridinderivate offenbart, wobei allgemein Angaben gemacht werden, dass diese Pteridine wertvolle pharmakologische Eigenschaften, nämlich coronarerweiternde, sedative, antipyretische und analgetische Wirkungen aufweisen.

Die aus dem Stand der Technik bekannten Phosphodiesterase 4 Inhibitoren sind bekannt dafür Nebenwirkungen wie Übelkeit und Erbrechen auszulösen (Doherty, 1999, Curr. Op. Chem. Biol., Aug. 3, (4);466-73). Die in dieser Erfindung benannten Substanzen sind besonders bevorzugt zur Behandlung der genannten Erkrankungen geeignet, da sie in einem Tiermodell für Übelkeit und Erbrechen (S. Murinus, Yamamoto K. et al.,Physiol. Behav., 2004, Oct. 30, 83(1), 151-6) diese Nebenwirkungen nicht auslösen.

Aufgabe der vorliegenden Erfindung ist es neue Verbindungen bereit zu stellen, die geeignet sind zur Vorbeugung oder Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems, oder Krebserkrankungen, insbesondere solche Verbindungen, die durch geringere Nebenwirkungen, insbesondere Emesis und Nausea, gekennzeichnet sind.

### BESCHREIBUNG DER ERFINDUNG

Überraschenderweise konnte nun gefunden werden, dass Pteridine der Formel **1** geeignet sind zur Behandlung entzündlicher Erkrankungen. Gegenstand der vorliegenden Erfindung sind deshalb Verbindungen der Formel **1** 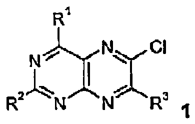 worin
- R¹: ein gesättigter oder ungesättigter, fünf-, sechs- oder siebengliedriger, heterocyclischer Ring, der ein Stickstoffatom und ein weiteres Atom ausgewählt aus der Gruppe bestehend aus Stickstoff, Schwefel und Sauerstoff enthalten kann;
- R²: ein fünf- , sechs- oder siebengliedriger, heterocyclischer Ring der ein Stickstoffatom und ein weiteres Atom ausgewählt aus der Gruppe bestehend aus Stickstoff, Schwefel und Sauerstoff enthalten kann;
- R³: eine Gruppe der Formel **1a**, 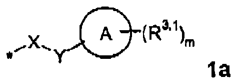
worin
- A: Aryl, wobei das Aryl für ein aromatisches Ringsystem mit 6 oder 10 Kohlenstoffatomen steht,;
- X: NR^{3.2},;
- Y: C₁₋₄-Alkylen, substituiert mit einem oder mehreren R^{3.3}
- m: 0, 1, 2;
- R^{3.1}: jeweils unabhängig voneinander C₁₋₄-Alkyl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₄-Haloalkyl, SO₂R^{3.1.1}, SO₂NH₂, Halogen, C₁₋₆-Haloalkyl, C₁₋₆-alkyl-CONH₂, O-C₁₋₆-alkyl-NH₂, O-C₃₋₆-Cycloalkyl, O-C₁₋₄-Alkylen-C₃₋₆-cycloalkyl oder Aryl, wobei das Aryl für ein aromatisches Ringsystem mit 6 oder 10 Kohlenstoffatomen steht; R^{3.1.1} H, C₁₋₄-Alkyl; R^{3.1.2} H, C₁₋₄-Alkyl; oder
- R^{3.1}: bildet gemeinsam mit zwei Atomen von A einen 5 oder 6-gliedrigen carbocyclischen Ring oder einen 5 oder 6-gliedrigen heterocyclischen Ring der ein oder mehrere Sauerstoff- oder Stickstoffatome enthalten kann;
- R^{3.2}: H, C₁₋₆-Alkyl;
- R^{3.3}: jeweils unabhängig voneinander C₁₋₆-Alkyl, C₁₋₆-Alkyl-OH, C₃₋₆-Cycloalkyl, C₃₋₆-Cycloalkyl-OH, O-C₁₋₆-Alkyl, COOH, COO-C₁₋₆-Alkyl, CONH₂;
- R^{3.3}: bildet gemeinsam mit einem oder zwei Kohlenstoffatomen von Y einen carbocyclischen Ring mit 3, 4, 5 oder 6 Kohlenstoffatomen,
wobei jedes zuvor genannte Aryl-Gruppe und auch jede zuvor genannte C₃₋₆-Cycloalkyl-Gruppe optional mit einem oder mehreren Resten ausgewählt aus der Gruppe bestehend aus Methyl, Ethyl, iso-Propyl, tert-Butyl; Hydroxy, Fluor, Chlor, Brom und Jod substituiert sein kann,
und wobei jeder zuvor genannte heterocyclische Ring optional mit einer Ketogruppe substituiert sein kann,
bedeuten, sowie pharmakologisch verträgliche Salz, Diastereomere, Enantiomere, Racemate, Hydrate oder Solvate davon.

Bevorzugt sind die obigen Verbindungen der Formel **1**, worin
- R¹: ein gesättigter oder ungesättigter, fünf- oder sechsgliedriger, heterocyclischer Ring, der ein Stickstoffatom und ein weiteres Atom ausgewählt aus der Gruppe bestehend aus Stickstoff und Schwefel enthalten kann;
- R²: ein fünf- oder sechsgliedriger, heterocyclischer Ring der ein oder zwei Stickstoffatome enthalten kann:
bedeuten, sowie pharmakologisch verträgliche Salz, Diastereomere, Enantiomere, Racemate, Hydrate oder Solvate davon.

Bevorzugt sind die obigen Verbindungen der Formel **1**, worin
- R¹: ein gesättigter oder ungesättigter, fünf- oder sechsgliedriger, heterocyclischer Ring, der ein Stickstoffatom und gegebenenfalls ein weiteres Schwefelatom enthält;
- R²: ein sechsgliedriger, heterocyclischer Ring der zwei Stickstoffatome enthält;
bedeuten, sowie pharmakologisch verträgliche Salz, Diastereomere, Enantiomere, Racemate, Hydrate oder Solvate davon.

Bevorzugt sind die obigen Verbindungen der Formel **1**, worin
- R³: eine Gruppe der Formel **1a**, worin
A Aryl;
X NR^{3.2};
Y C₁₋₄-Alkylen, substituiert mit einem oder mehreren R^{3.3}
m 0, 1, 2;
R^{3.1} jeweils unabhängig voneinander C₁₋₄-Alkyl, COOR^{3.1.1}, CONR^{3.1.1} R^{3.1.2}, CN, NR^{3.1.1}R^{3.1.2}, NHCOR^{3.1.1}, OR^{3.1.1}, O-C₁₋₄-Haloalkyl, SO₂R^{3.1.1}, SO₂NH₂, Halogen, C₁₋₆-Haloalkyl, C₁₋₆-alkyl-CONH₂, O-C₁₋₆-alkyl-NH₂, O-C₃₋₆-Cycloalkyl, O-C₁₋₄-Alkylen-C₃₋₆-cycloalkyl oder Aryl, wobei das Aryl für ein aromatisches Ringsystem mit 6 oder 10 Kohlenstoffatomen steht;
R^{3.1.1} H, C₁₋₄-Alkyl;
R^{3.1.2} H, C₁₋₄-Alkyl;
R^{3.2} H, C₁₋₆-Alkyl;
R^{3.3} jeweils unabhängig voneinander C₁₋₆-Alkyl, C₁₋₆-Alkyl-OH, C₃₋₆-Cycloalkyl, C₃₋₆-Cycloalkyl-OH, O-C₁₋₆-Alkyl, COOH, COO-C₁₋₆-Alkyl, CONH₂;
R^{3.3} bildet gemeinsam mit einem oder zwei Kohlenstoffatomen von Y einen carbocyclischen Ring mit 3, 4, 5 oder 6 Kohlenstoffatomen
bedeutet, sowie pharmakologisch verträgliche Salz, Diastereomere, Enantiomere, Racemate, Hydrate oder Solvate davon.

Bevorzugt sind die obigen Verbindungen der Formel **1**, worin
- R³: ist eine Gruppe der allgemeinen Formel **1a**, worin
A Phenyl;
X NR^{3.2}, S, O;
Y C₁₋₄-Alkylen, substituiert mit einem oder mehreren R^{3.3}
m 0, 1 oder 2;
R^{3.1} jeweils unabhängig voneinander C₁₋₄-Alkyl, COOR^{3.1.1}, CONR^{3.1.1}R^{3.1.2}, CN, NR^{3.1.1}R^{3.1.2}, NHCOR^{3.1.1} OR^{3.1.1}, O-C₁₋₄-Haloalkyl, SO₂R^{3.1.1}, SO₂NH₂, Halogen oder Aryl, wobei das Aryl für ein aromatisches Ringsystem aus 6 oder 10 Kohlenstoffatomen steht;
R^{3.1.1} H, C₁₋₆-Alkyl;
R^{3.1.2} H, C₁₋₆-Alkyl;
R^{3.2} H, C₁₋₆-Alkyl;
R^{3.3} jeweils unabhängig voneinander C₁₋₆-Alkyl, C₁₋₆-Alkyl-OH, C₃₋₆-Cycloalkyl, O-C₁₋₆-Alkyl, COOH, COO-C₁₋₆-Alkyl, CONH₂;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

Bevorzugt sind die obigen Verbindungen der Formel **1**, worin
- R³: ist eine Gruppe der allgemeinen Formel **1a**, worin
A Phenyl;
X NR^{3.2;}
Y C₁₋₂-Alkylen, substituiert mit einem oder mehreren R^{3.3}
m 0, 1 oder 2;
R^{3.1} jeweils unabhängig voneinander C₁₋₄-Alkyl, Aryl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₄-Haloalkyl, SO₂R^{3.1.1}, SO₂NH₂, Halogen;
R^{3.1.1} H, C₁₋₄-Alkyl;
R^{3.1.2} H, C₁₋₄-Alkyl;
- R^{3.2}: H, C₁₋₄-Alkyl:
- R^{3.3}: jewells unabhängig voneinander C₁₋₄-Alkyl, C₁₋₄-Alkyl-OH, C₃₋₆-Cycloalkyl, O-C₁₋₄-Alkyl, COOH, COO-C₁₋₄-Alkyl, CONH₂;
- R^{3.3}: bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

Bevorzugt sind die obigen Verbindungen der Formel **1**, worin
- R³: ist eine Gruppe der allgemeinen Formel **1a**, worin
A Phenyl;
X NR^{3.2};
Y C₁₋₂Alkylen, substituiert mit einem oder mehreren R^{3.3}
m 0, 1 oder 2;
R^{3.1} jeweils unabhängig voneinander C₁₋₄-Alkyl, Aryl, COOH, COO-C₁₋₄-Alkyl, CONH₂, CN, NH₂, NHCO-C₁₋₄-Alkyl, OH, O-C₁₋₄-Alkyl, O-C₁₋₄-Haloalkyl, SO₂-C₁₋₄-Alkyl, SO₂NH₂, Halogen;
R^{3.2} H, C₁₋₄-Alkyl;
R^{3.3} jeweils unabhängig voneinander C₁₋₄-Alkyl, C₁₋₄-AlKyl-OH, C₃₋₆-Cycloalkyl, O-C₁₋₄-Alkyl, COOH, COO-C₁₋₄-Alkyl, CONH₂;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

Bevorzugt sind die obigen Verbindungen der Formel **1**, worin
- R³: ist eine Gruppe der allgemeinen Formel **1a**, worin
A Phenyl;
X NR^{3.2};
Y C₁₋₂-Alkylen, substituiert mit einem oder mehreren R^{3.3}
m 0, 1 oder 2;
R^{3.1} jeweils unabhängig voneinander Methyl, Ethyl, Propyl, Ph, COOH, COOMe, CONH₂, CN, NH₂, NHCOMe, OH, OMe, OEt, OCF₃, OCHF₂, SO₂Me, SO₂NH₂, F, Cl, Br;
R^{3.2} H, C₁₋₄-Alkyl;
R^{3.3} jeweils unabhängig voneinander Methyl, Ethyl, Propyl, Butyl, CH₂OH, CH₂CH₂OH, C(CH₂)₂OH, Cyclopropyl, COOH, COOMe, COOEt, COOPr CONH₂, OMe, OEt, OPr,
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

Ebenfalls bevorzugt sind die obigen Verbindungen der Formel **1**, worin
- R³: ist eine Gruppe der allgemeinen Formel **1a**, worin
A Phenyl;
X NR^{3.2.};
Y C₁₋₂-Alkylen, substituiert mit einem oder mehreren R^{3.3}
m 0, 1 oder 2;
R^{3.1} jeweils unabhängig voneinander C₁₋₄-Alkyl, CN, O-C₁₋₄-Alkyl, Halogen;
R^{3.2} H;
R^{3.3} jeweils unabhängig voneinander C₁₋₄-Alkyl, C₁₋₄-Alkyl-OH, C₃₋₆-Cycloalkyl, COOH, COO-C₁₋₄-Alkyl, CONH₂, O-C₁₋₄-Alkyl;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

Bevorzugt sind die obigen Verbindungen der Formel **1**, worin
- R³: ist eine Gruppe der allgemeinen Formel **1a**, worin
A Phenyl;
X NR^{3.2};
Y C₁₋₂-Alkylen, substituiert mit einem oder mehreren R^{3.3}
m 0, 1 oder 2;
R^{3.1} jeweils unabhängig voneinander Methyl, iso-Propyl, OMe, F, Cl, Br, CN,
R^{3.2} H;
R^{3.3} jeweils unabhängig voneinander Methyl, Cyclopropyl, CH₂OH, CH₂CH₂OH, C(CH₂)₂OH, COOH, COOMe, CONH₂, OMe,
R^{3.3} bildet gemeinsam mit einem oder zwei Kohlenstoffatomen von Y einen carbocyclischen Ring mit 3 Kohlenstoffatomen
bedeutet, sowie pharmakologisch verträgliche Salz, Diastereomere, Enantiomere, Racemate, Hydrate oder Solvate davon.

Insbesondere bevorzugt sind die obigen Verbindungen der Formel **1**, worin
- R¹: Pyrrolidinyl, 2,5-Dihydro-1H-pyrrolyl, Thiomorpholinyl;
- R²: Piperazinyl;
- R³: ein Rest ausgewählt aus der Gruppe bestehend aus 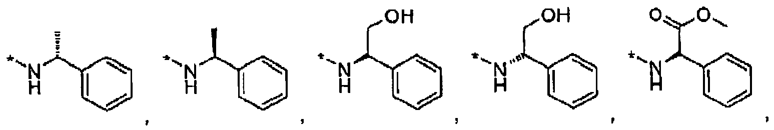 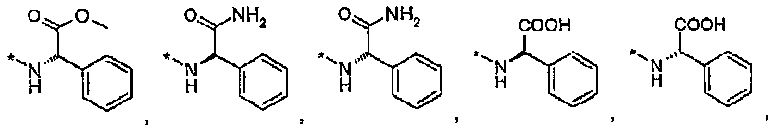 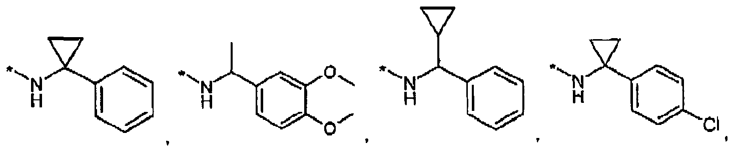 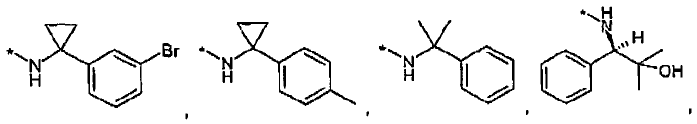 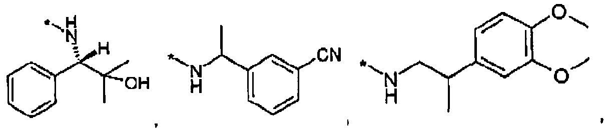 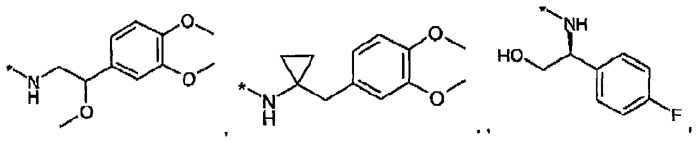 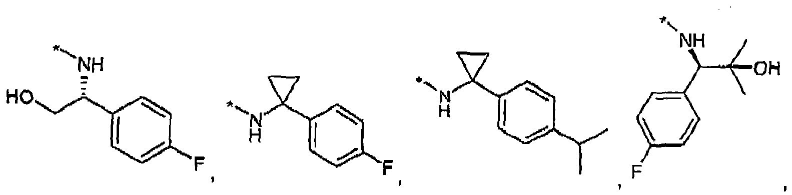 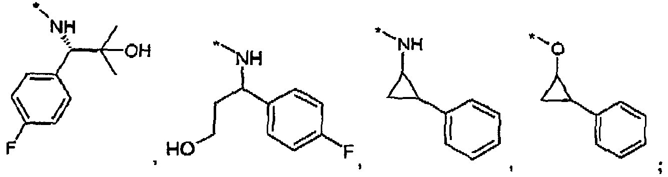
bedeuten, sowie pharmakologisch verträgliche Salz, Diastereomere, Enantiomere, Racemate, Hydrate oder Solvate davon.

### VERWENDETE BEGRIFFE UND DEFINITIONEN

Im Rahmen dieser Anmeldung Können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. So werden zum Beispiel die Reste N-Piperidinyl (**I**), 4-Piperidinyl (**II**), 2-Tolyl (**III**), 3-Tolyl (**IV**) und 4-Tolyl (**V)** wie folgt dargestellt:

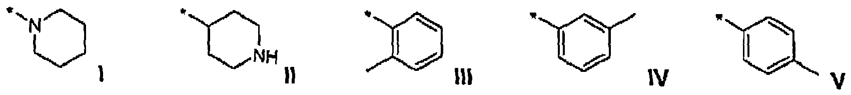

Befindet sich in der Strukturformel des Substituenten kein Stern (*), so kann an dem Substituenten jedes Wasserstoffatom entfernt werden und die dadurch freiwerdende Valenz als Bindungsstelle zum Rest eines Molekül dienen. So kann zum Beispiel **VI** die Bedeutung von 2-Tolyl, 3-Tolyl, 4-Tolyl und Benzyl haben.

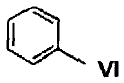

Unter pharmakologisch verträglichen Säureadditionssalzen werden beispielsweise diejenigen Salze verstanden, die ausgewählt sind aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n*-Propyl, *iso*-Propyl, *n-*Butyl, *iso*-Butyl, *sec*-Butyl, *tert*-Butyl, *n*-Pentyl, *iso-*Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₄-Alkylen" (auch soweit sie Bestandteil anderer Reste sind) werden verzweigte und unverzweigte Alkylengruppen mit 1 bis 4 Kohlenstoffatomen verstanden. Beispielsweise werden hierfür genannt: Methylen, Ethylen, Propylen, 1-Methylethylen, Butylen, 1-Methylpropylen, 1,1-Dimethylethylen oder 1,2-Dimethylethylen. Sofern nicht anders beschrieben, umfassen die Definitionen Propylen und Butylen alle denkbaren isomeren Formen der jeweiligen Reste gleicher Kohlenstoffanzahl. So umfasst beispielsweise Propyl auch 1-Methylethylen und Butylen umfasst 1-Methylpropylen; 1,1-Dimethylethylen, 1,2-Dimethylethylen. Sollte die Kohlenstoffkette mit einem Rest substituiert sein, der gemeinsam mit einem oder zwei Kohlenstoffatomen der Alkylenkette einen carbocyclischen Ring mit 3, 4, 5 oder 6 Kohlenstoffatomen bildet, so sind damit folgende Beispiele der Ringe umfasst:

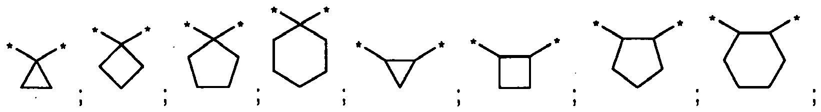

Unter dem Begriff "C₃₋₆-Cycloalkyl" (auch soweit sie Bestandteil anderer Reste sind) werden cyclische Alkylgruppen mit 3 bis 6 Kohlenstoffatomen verstanden. Beispielsweise werden hierfür genannt: Cyclopropyl, Cyclobutyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso-*Propyl, *tert*-Butyl, Hydroxy, Fluor, Chlor, Brom und Jod.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "heterocyclische Ringe" oder "Het" werden fünf-, sechs- oder siebengliedrige, gesättigte oder ungesättigte heterocyclische Ringe oder 5-10 gliedrige, bicyclische Heteroringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Als Beispiele für fünf-, sechs- oder siebengliedrige, gesättigte oder ungesättigte heterocyclische Ringe, werden genannt:

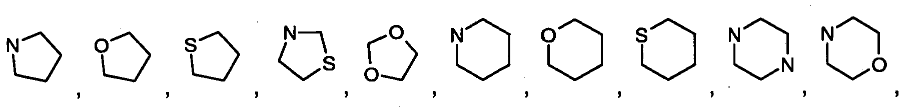

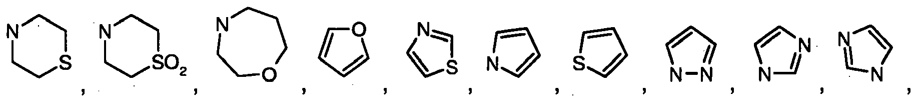

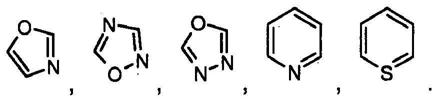

Soweit nicht anders erwähnt, kann ein heterocyclischer Ringe mit einer Ketogruppe versehen sein. Als Beispiel hierfür werden genannt.

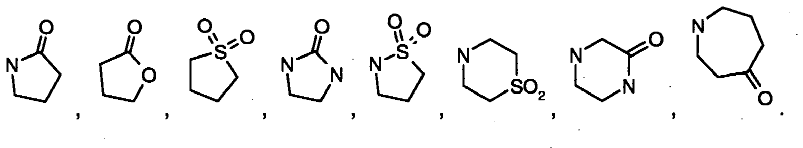

### BEISPIELE

Die erfindungsgemäßen Verbindungen können nach an sich aus der Literatur bekannten Methoden hergestellt werden, wie sie zum Beispiel in DE 3540952 beschrieben sind. Weitere Herstellvarianten für die unten gezeigten Verbindungen sind im folgenden aufgeführt.

**Verbindung 12:**
a) 1-Phenyl-cyclopropancarbonylchlorid: 7,00 g (41,87 mmol) 1-Phenylcyclopropancarbonsäure, 30,45 ml (418,70 mmol) Thionylchlorid und 1 Tropfen Dimethylformamid werden in 100 ml Dichlormethan vorgelegt, dann 3 Stunden unter Rückfluss gerührt. Anschließend wird das Reaktionsgemisch eingedampft, in Toluol aufgenommen und wieder eingedampft. Der Rückstand wird mit Wasser und Dichlormethan versetzt und extrahiert. Die organische Phase wird mit Wasser gewaschen, getrocknet und zur Trockene eingedampft. Ausbeute: 7,53 g
b) 1-Phenyl-cyclopropylamin: 7,53 g (41,69 mmol) 1-Phenyl-cyclopropancarbonylchlorid werden in 50 ml Xylol vorgelegt, 3,25 g (50,03 mmol) Natriumazid zugegeben. Das Reaktionsgemisch wird erst auf 80° C, nach 1 Stunde auf 110°C erhitzt und 1 Stunde weiter gerührt. Nach Abkühlen auf Raumtemperatur wird filtriert und zum Filtrat konz. Salzsäure gegeben. Es wird auf 70° C erhitzt bis die CO₂-Entwicklung beendet ist, dann 1 Stunde bei 100° C gerührt. Anschließend wird das Reaktionsgemisch mit 4 N Salzsäure extrahiert, die wässrige Phase alkalisch gestellt und mit Petrolether extrahiert. Die organische Phase wird getrocknet und zur Trockene eingedampft. Ausbeute: 1,07 g (= 19% d. Th.)
c) (6-Chloro-2-piperazin-1-yl-4-pyrrolidin-1-yl-pteridin-7-yl)-(1-phenyl-cyclopropyl)-amin *(Beispiel 12):* 80 mg (0,263 mmol) 2,6,7-Trichlor-4-pyrrolidin-1-yl-pteridin werden in 5 ml Dioxan gelöst, 0,064 ml (0,368 mmol) Diisopropylethylamin und 35 mg (0,263 mmol) 1-Phenyl-cyclopropylamin zugegeben. Das Reaktionsgemisch wird 16 Stunden bei Raumtemperatur und 24 Stunden bei 40°C gerührt. 64,14 mg (0,344 mmol) Piperazin und 0,064 ml (0,368 mmol) Diisopropylethylamin werden zugegeben, 16 Stunden bei 100° C gerührt. Anschließend wird das Reaktionsgemisch eingedampft, 15 ml 50%ige Trifluoeressigsäure in Dichlormethan zugegeben und 3 Stunden bei Raumtemperatur gerührt. Das Reaktionsgemisch wird eingedampft, der Rückstand mit Dichlormethan und 1 N Natronlauge extrahiert. Die vereinten organischen Phasen werden getrocknet und zur Trockene eingedampft. Der Rückstand wird chromatographisch gereinigt. Ausbeute: 21 mg (=18% d. Th.)

**Verbindung 13:**
a) 1-(3,4-Dimethoxy-phenyl)-ethylamin: 37,00 ml (111 mmol) Methylmagnesiumbromid in Diethylether werden vorgelegt, eine Lösung aus 6,00 g (36,40 mmol) 3,4-Dimethoxy-benzonitril in 50 ml Tetrahydrofuran unter Eiskühlung zugetropft, dann 3 Stunden unter weiterer Kühlung gerührt. Nach Zugabe von 0,82 eq Methylmagnesiumbromidlösung wird 1,5 Stunden nachgerührt. Anschließend werden 120 ml Methanol zugetropft, danach 2,78 g (72,80 mmol) Natriumborhydrid portionsweise zugegeben. Das Reaktionsgemisch wird 16 Stunden bei Raumtemperatur gerührt, danach im Vakuum konzentriert, mit Wasser und Chloroform versetzt. Das Gemisch wird auf pH 1 gestellt, die Phasen getrennt. Die wässrige Phase wird mit Chloroform extrahiert, dann alkalisch gestellt und erneut mit Chloroform extrahiert. Die organische Phase wird getrocknet und zur Trockene eingedampft. Ausbeute: 1,71 g (= 26% d. Th.)
b) (6-Chloro-2-piperazin-1-yl-4-pyrrolidin-1-yl-pteridin-7-yl)-[1-(3,4-dimethoxy-phenyl)-ethyl]-amin (*Beispiel 13*): 80 mg (0,263 mmol) 2,6,7-Trichlor-4-pyrrolidin-1-yl-pteridin werden in 5 ml Dioxan gelöst, 0,064 ml (0,368 mmol) Diisopropylethylamin und 48 mg (0,265 mmol) 1-(3,4-Dimethoxy-phenyl)-ethylamin zugegeben. Das Reaktionsgemisch wird 72 Stunden bei 40°C gerührt. 113 mg (1,31 mmol) Piperazin werden in 10 ml Dioxan gelöst, auf 80°C erhitzt und das Reaktionsgemisch zugetropft. Es wird 2 Stunden gerührt, anschließend wird das Reaktionsgemisch in 20 ml Eiswasser getropft und mit Dichlormethan extrahiert. Die organische Phase wird getrocknet und zur Trockene eingedampft. Der Rückstand wird chromatographisch gereinigt, entsprechende Fraktion eingedampft. Ausbeute: 73 mg (= 56% d. Th.)

**Verbindung 14:**
a) C-Cyclopropyl-C-phenyl-methylamin: 60,00 ml (30 mmol) Cyclopropylmagnesiumbromid in Tetrahydrofuran werden vorgelegt, eine Lösung aus 1,10 ml (10,22 mmol) Benzonitril in 15 ml Tetrahydrofuran unter Eisbadkühlung zugetropft. Es wird 5,5 Stunden unter weiterer Kühlung gerührt. Danach werden 30 ml Methanol zugetropft und 800 mg (20,94 mmol) Natriumborhydrid portionsweise zugegeben. Das Reaktionsgemisch wird 16 Stunden bei Raumtemperatur gerührt, dann eingedampft. Der Rückstand wird mit Chloroform und Wasser versetzt, auf pH 1 gestellt und die Phasen getrennt. Die wässrige Phase wird mit Chloroform extrahiert, dann alkalisch gestellt und erneut mit Chloroform extrahiert. Die daraus resultierende organische Phase wird getrocknet und zur Trockene eingedampft. Ausbeute: 1,38 g (= 92% d. Th.)
b) C-(6-Chloro-2-piperazin-1-yl-4-pyrrolidin-1-yl-pteridin-7-yl)-C-cyclopropyl-C-phenyl-methylamin *(Beispiel 14)*: 80 mg (0,263 mmol) 2,6,7-Trichlor-4-pyrrolidin-1-yl-pteridin werden in 5 ml Dioxan gelöst, 0,064 ml (0,368 mmol) Diisopropylethylamin und 38,67 mg (0,263 mmol) 1-Phenyl-cyclopropylamin zugegeben. Das Reaktionsgemisch wird 72 Stunden bei Raumtemperatur gerührt. 113 mg (1,31 mmol) Piperazin werden in 10 ml Dioxan gelöst, auf 80°C erhitzt und das Reaktionsgemisch zugetropft. Es wird 2 Stunden gerührt, anschließend wird das Reaktionsgemisch in 20 ml Eiswasser getropft und mit Dichlormethan extrahiert. Die organische Phase wird getrocknet und zur Trockene eingedampft. Der Rückstand wird chromatographisch gereinigt, entsprechende Fraktion eingedampft und mit Diethylether/Petrolether verrieben. Ausbeute: 47,70 mg (= 39% d. Th.)

**Verbindung 15:**
a) 1-(4-Chlorphenyl)-cyclopropylamin: 500 mg (2,54 mmol) 1-(4-Chlorphenyl)-cyclopropan-carbonsäure, 1,20 ml (5,38 mmol) Phosphorsäurediphenylesterazid und 0,39 ml (2,80 mmol) Triethylamin werden in 10 ml Dimethylformamid vorgelegt, dann 16 Stunden bei Raumtemperatur gerührt. Anschließend wird das Reaktionsgemisch bei 100° C innerhalb 2 Stunden in 50 ml Wasser und 10 ml 1 N Salzsäure getropft, abgekühlt und mit Natronlauge neutralisiert. Der ausgefallene Niederschlag wird abgesaugt, das Filtrat mit Ethylacetat extrahiert. Die organische Phase wird getrocknet und zur Trockene eingedampft. Der Rückstand wird sauer extrahiert, die wässrige Phase neutralisiert und mit Dichlormethan extrahiert. Die daraus resultierende organische Phase wird getrocknet und zur Trockene eingedampft. Ausbeute: 145 mg (= 34% d. Th.)
b) [1-(4-Chlorphenyl)-cyclopropyl]-(6-Chloro-2-piperazin-1-yl-4-pyrrolidin-1-yl-pteridin-7-yl)-amin *(Beispiel 15)*: 80 mg (0,263 mmol) 2,6,7-Trichlor-4-pyrrolidin-1-yl-pteridin werden in 5 ml Dioxan gelöst, 0,064 ml (0,368 mmol) Diisopropylethylamin und 44 mg (0,263 mmol) 1-(4-Chlorphenyl)-cyclopropylamin zugegeben, dann auf 40° C erhitzt. Das Reaktionsgemisch wird 24 Stunden gerührt, nochmals 1 Äquivalent 1-(4-Chlorphenyl)-cyclopropylamin zugegeben und weitere 24 Stunden bei 40° C gerührt. Anschließend wird das Reaktionsgemisch über Kieselgel filtriert und eingedampft. 113 mg (1,31 mmol) Piperazin werden in 15 ml Dioxan gelöst, auf 80° C erhitzt und das Reaktionsgemisch zugetropft. Es wird 1 Stunde gerührt, anschließend wird das Reaktionsgemisch in 20 ml Eiswasser getropft und mit Dichlormethan extrahiert. Die organische Phase wird getrocknet und zur Trockene eingedampft. Der Rückstand wird chromatographisch gereinigt, entsprechende Fraktion eingedampft und mit Petrolether/Diethylether verrieben. Ausbeute: 108 mg (= 85% d. Th.)

**Verbindung 16:**
a) 1-(3-Bromphenyl)-cyclopropylamin: 500 mg (2,07 mmol) 1-(3-Bromphenyl)-cyclopropan-carbonsäure, 0,46 ml (2,07 mmol) Phosphorsäurediphenylesterazid und 0,32 ml (2,28 mmol) Triethylamin werden in 10 ml Dimethylformamid vorgelegt, dann 16 Stunden bei Raumtemperatur gerührt. Anschließend wird das Reaktionsgemisch bei 100 °C innerhalb 2 Stunden in 50 ml Wasser und 10 ml 1 N Salzsäure getropft, abgekühlt und mit Natronlauge neutralisiert. Der ausgefallene Niederschlag wird abgesaugt, das Filtrat mit Ethylacetat extrahiert. Die organische Phase wird getrocknet und zur Trockene eingedampft. Ausbeute: 332 mg (= 75% d. Th.)
b) [1-(3-Bromphenyl)-cyclopropyl]-(6-Chloro-2-piperazin-1-yl-4-pyrrolidin-1-yl-pteridin-7-yl)-amin *(Beispiel 16)*: 80 mg (0,263 mmol) 2,6,7-Trichlor-4-pyrrolidin-1-yl-pteridin werden in 5 ml Dioxan gelöst, 0,064 ml (0,368 mmol) Diisopropylethylamin und 56 mg (0,263 mmol) 1-(3-Bromphenyl)-cyclopropylamin zugegeben, dann auf 40°C erhitzt. Das Reaktionsgemisch wird 72 Stunden gerührt.113 mg (1,31 mmol) Piperazin werden in 15 ml Dioxan gelöst, auf 80° C erhitzt und das Reaktionsgemisch zugetropft. Es wird 1 Stunde gerührt, anschließend wird das Reaktionsgemisch in 20 ml Eiswasser getropft und mit Dichlormethan extrahiert. Die organische Phase wird getrocknet und zur Trockene eingedampft. Der Rückstand wird chromatographisch gereinigt, entsprechende Fraktion eingedampft und mit Petrolether/Diethylether verrieben. Ausbeute: 125 mg (= 90% d. Th.)

**Verbindung 17:**
a) 1-p-Tolyl-cyclopropylamin: 500 mg (2,84 mmol) 1-p-Tolylcyclopropan-carbonsäure, 0,63 ml (2,84 mmol) Phosphorsäurediphenylesterazid und 0,40 ml (2,84 mmol) Triethylamin werden in 10 ml Dimethylformamid vorgelegt, dann 16 Stunden bei Raumtemperatur gerührt. Anschließend wird das Reaktionsgemisch bei 100° C innerhalb 2 Stunden in 50 ml Wasser und 10 ml 1 N Salzsäure getropft, abgekühlt und mit Natronlauge neutralisiert. Der ausgefallene Niederschlag wird abgesaugt, das Filtrat mit Ethylacetat extrahiert. Die organische Phase wird getrocknet und zur Trockene eingedampft. Der Rückstand wird sauer extrahiert, die wässrige Phase neutralisiert und mit Dichlormethan extrahiert. Die daraus resultierende organische Phase wird getrocknet und zur Trockene eingedampft. Ausbeute: 110 mg (= 26% d. Th.)
b) (6-Chloro-2-piperazin-1-yl-4-pyrrolidin-1-yl-pteridin-7-yl)-(1p-tolyl-cyclopropyl)-amin *(Beispiel 17):* 80 mg (0,263 mmol) 2,6,7-Trichlor-4-pyrrolidin-1-yl-pteridin werden in 5 ml Dioxan gelöst, 0,064 ml (0,368 mmol) Diisopropylethylamin und 43 mg (0,292 mmol) 1-p-Tolyl-cyclopropylamin zugegeben, dann auf 40°C erhitzt. Das Reaktionsgemisch wird 72 Stunden gerührt.113 mg (1,31 mmol) Piperazin werden in 15 ml Dioxan gelöst, auf 80° C erhitzt und das Reaktionsgemisch zugetropft. Es wird 1 Stunde gerührt, anschließend wird das Reaktionsgemisch in 20 ml Eiswasser getropft und mit Dichlormethan extrahiert. Die organische Phase wird getrocknet und zur Trockene eingedampft. Der Rückstand wird chromatographisch gereinigt, entsprechende Fraktion eingedampft und mit Petrolether/Diethylether verrieben. Ausbeute: 80 mg (= 65% d. Th.)

**Verbindung 20:**
a) (S)-Phenyl-(2,2,2-trifluoro-acetylamino)-essigsäuremethylester: 3,00 g (14,877 mmol) (S)-Phenylglycinmethylester hydrochlorid und 2,48 ml (17,853 mmol) Triethylamin werden in 25 ml Tetrahydrofuran vorgelegt und auf -78° C gekühlt. 2,50 ml (18 mmol) Trifluoressigsäureanhydrid werden langsam zugetropft. Nach Wegnahme der Kühlung wird das Reaktionsgemisch 16 Stunden bei Raumtemperatur gerührt. Anschließend wird mit Wasser versetzt, dann mit Ethylacetat extrahiert. Die vereinigten organischen Phasen werden gewaschen, getrocknet und zur Trockene eingedampft. Ausbeute: 3,90 g (=100% d. Th.)
b) (S)-2,2,2-Trifluor-N-(2-hydroxy-2-methyl-1-phenyl-propyl)-acetamid: 1,00 g (3,829 mmol) (S)-Phenyl-(2,2,2-trifluoro-acetylamino)-essigsäuremethylester werden in 40 ml Diethylether vorgelegt, 3,83 ml (11,486 mmol) Methylmagnesiumjodid-Lösung langsam zugetropft. Die Temperatur sollte 20 ° C nicht übersteigen. Die Suspension wird 16 Stunden bei Raumtemperatur gerührt, dann auf Eiswasser gegossen . Es erfolgt Zugabe von Ammoniumchlorid bis der Niederschlag gelöst ist. Die wässrige Phase wird mit Diethylether extrahiert, die vereinigten organischen Phasen getrocknet und zur Trockene eingedampft. Ausbeute: 1,20 g (>100% d. Th)
c) (S)-1-Amino-2-methyl-1-phenyl-propan-2-ol: 1,20 g (4,593 mmol) (S)-2,2,2-Trifluor-N-(2-hydroxy-2-methyl-1-phenyl-propyl)-acetamid und 0,515 g (9,187 mmol) Kaliumhydroxid werden in 15 ml Methanol vorgelegt und 16 Stunden bei 60°C gerührt. Anschließend wird das Reaktionsgemisch mit Wasser versetzt und mit Dichlormethan extrahiert. Die organischen Phasen werden vereinigt, getrocknet und zur Trockene eingedampft. Ausbeute: 500 mg (= 53% d. Th.)
d) (S)-1-(6-Chloro-2-piperazin-1-yl-4-pyrrolidin-1-yl-pteridin-7-ylamino)-2-methyl-1-phenyl-propan-2-ol *(Beispiel 20)*: 100 mg (0,279 mmol) (S)-2,6,7-Trichlor-4-pyrrolidin-1-yl-pteridin werden in 15 ml Dioxan gelöst, 0,053 ml (0,307 mmol) Diisopropylethylamin und 51,24 mg (0,279 mmol) 1-Amino-2-methyl-1-phenyl-propan-2-ol zugegeben, dann auf 40 ° C erhitzt. Das Reaktionsgemisch wird 16 Stunden gerührt. Es werden noch 0,4 eq 1-Amino-2-methyl-1-phenyl-propan-2-ol zugegeben, 3 Stunden bei 40° C gerührt. 120 mg (1,40 mmol) Piperazin werden in 5 ml Dioxan gelöst, auf 80°C erhitzt und das Reaktionsgemisch zugetropft. Es wird 16 Stunden gerührt, anschließend wird die Reaktionslösung im Vakuum konzentriert und in 50 ml Eiswasser getropft. Der ausfallende Niederschlag wird abgesaugt und chromatographisch gereinigt. Entsprechende Fraktionen werden vereinigt, zur Trockene eingedampft. Der Rückstand wird in Dioxan aufgenommen und gefriergetrocknet. Ausbeute: 45 mg (= 33% d. Th.)

**Verbindung 21:**
a) 3-(1-Amino-ethyl)-benzonitril: 9,40 g (64,757 mmol) 3-Cyano-Acetophenon, 40,00 g (518,941 mmol) Ammoniumacetat und 10,00 g (186,951 mmol) Ammoniumchlorid werden in Methanol vorgelegt,16 Stunden bei 40°C gerührt. 2,90 g (46,149 mmol) Natriumcyanoborhydrid werden zugegeben, dann wieder 16 Stunden gerührt. Das Reaktionsgemisch wird mit Eisessig auf pH3 eingestellt, dann das Methanol eingedampft. Beim Abkühlen fällt ein Niederschlag aus. Dieser wird abgesaugt. Das Filtrat wird mit konz. Natronlauge alkalisch gestellt, er dabei ausfallende Niederschlag abgesaugt. Das Filtrat wird mit Diethylether extrahiert, die vereinten organischen Phasen getrocknet und zur Trockene eingedampft. Der Rückstand wird per Vakuumdestillation gereinigt. Ausbeute: 1,10 g (= 12% d. Th.)
b) 3-[1-(6-Chloro-2-piperazin-1-yl-4-pyrrolidin-1-yl-pteridin-7-ylamino)-ethyl]-benzonitril *(Beispiel 21):* 90,00 mg (0,296 mmol) 2,6,7-Trichlor-4-pyrrolidin-1-yl-pteridin und 0,057 ml (0,304 mmol) Diisopropylethylamin werden in 10 ml Dioxan gelöst, eine Lösung aus 43,20 mg (0,296 mmol) 3-(1-Amino-ethyl)-benzonitril in 5 ml Dioxan bei Raumtemperatur zugetropft. Das Reaktionsgemisch wird 2 Stunden bei Raumtemperatur und 16 Stunden bei 40° C gerührt, dabei werden noch 1 eq Diisopropylethylamin und 3-(1-Amino-ethyl)-benzonitril zugesetzt. 127 mg (1,47 mmol) Piperazin werden in 20 ml Dioxan gelöst, auf 80° C erhitzt und das Reaktionsgemisch zugetropft. Es wird 16 Stunden gerührt, anschließend wird die Reaktionslösung im Vakuum konzentriert und in 50 ml Eiswasser getropft. Der ausfallende Niederschlag wird abgesaugt und chromatographisch gereinigt. Entsprechende Fraktionen werden vereinigt, zur Trockene eingedampft. Der Rückstand wird in Dioxan aufgenommen und gefriergetrocknet. Ausbeute: 30,0 mg (= 22% d. Th.)

**Verbindung 24:**
a) 1-(3,4-Dimethoxy-benzyl)-cyclopropylamin: 5,20 g (28,76 mmol) 3,4-Dimethoxybenzylcyanid werden in 150 ml Diethylether vorgelegt und 9,00 ml (30,708 mmol) Titanium(IV)isopropoxid zugegeben. Unter Eiskühlung werden 20,00 ml (60 mmol) 3 molare Ethylmagnesiumbromid-Lösung werden zugetropft, dann 0,5 Stunden nachgerührt. Danach werden 7,60 ml (59,97 mmol) Bortrifluoridetherat zugetropft und 0,5 Stunden nachgerührt. Anschließend wird das Reaktionsgemisch unter Kühlung mit 90 ml 1 N Natronlauge versetzt, 1 Stunde bei Raumtemperatur nachgerührt. Die organische Phase wird abgetrennt, die wässrige Phase mit Diethylether extrahiert. Die vereinten organischen Phasen werden mit gesättigter Natriumsulfonatlösung gewaschen und sauer extrahiert. Die daraus resultierende wässrige Phase wird mit Dichlormethan extrahiert, die organischen Extrakte getrocknet und zur Trockene eingedampft. Der Rückstand wird chromatographisch gereinigt. Ausbeute: 1,60 g (=27% d. Th.)
b) (6-Chloro-2-piperazin-1-yl-4-pyrrolidin-1-yl-pteridin-7-yl)-[1-(3,4-dimethoxybenzyl)-cyclopropyl]-amin *(Beispiel 24)*: 80 mg (0,263 mmol) 2,6,7-Trichlor-4-pyrrolidin-1-yl-pteridin werden in 5 ml Dioxan suspendiert, 54 mg (0,260 mmol) 1-(3,4-Dimethoxybenzyl)-cyclopropylamin und 0,064 ml (0,368 mmol) Diisopropylethylamin zugegeben. Es wird 72 Stunden bei 40 ° C gerührt. 113 mg (1,31 mmol) Piperazin werden in 15 ml Dioxan gelöst, auf 80° C erhitzt und das Reaktionsgemisch zugetropft. Es wird 2 Stunden gerührt, anschließend in 20 ml Eiswasser getropft. Es wird mit Dichlormethan extrahiert, die organische Phase getrocknet und zur Trockene eingedampft. Der Rückstand wird chromatographisch gereinigt. Entsprechende Fraktion wird zur Trockene eingedampft, dann mit Petrolether/Diethylether verrieben. Ausbeute: 78 mg (= 57% d. Th.)

**Verbindung 27:**
a) 1-(4-Fluorphenyl)-cyclopropylamin: 5,00 g (41,28 mmol) 4-Fluorbenzonitril und 12,10 g (41,28 mmol) Titanium(IV)isopropoxid werden in 100 ml Diethylether vorgelegt und auf 70° C gekühlt. 30,28 ml (90,83 mmol) 3 molare Ethylmagnesiumbromid-Lösung werden zugetropft, dann wird 0,1 Stunden nachgerührt. Nach Erwärmen auf Raumtemperatur werden 10,42 ml (82,56 mmol) Bortrifluoridetherat zugetropft und 1 Stunde nachgerührt. Anschließend wird das Reaktionsgemisch mit 56 ml 1 N Salzsäure versetzt, danach werden 80 ml 4 N Natronlauge zugegeben. Der dabei ausfallende Niederschlag wird abgesaugt und verworfen. Das Filtrat wird mit Wasser extrahiert, die organischen Phasen vereinigt, getrocknet und zur Trockene eingedampft. Der Rückstand wird in Dichlormethan aufgenommen, sauer extrahiert und neutralisiert. Die organische Phase wird getrocknet und zur Trockene eingedampft. Der Rückstand wird chromatographisch gereinigt. Ausbeute: 1,818 g (=29% d. Th.)
b) (6-Chloro-2-piperazin-1-yl-4-pyrrolidin-1-yl-pteridin-7-yl)-[1-(4-fluorphenyl)-cyclopropyl]-amin *(Beispiel 27)*:80 mg (0,263 mmol) 2,6,7-Trichlor-4-pyrrolidin-1-yl-pteridin werden in 5 ml Dioxan suspendiert, 40 mg (0,264 mmol) 1-(4-Isopropyl-phenyl)-cyclopropylamin und 0,064 ml (0,368 mmol) Diisopropylethylamin werden zugegeben. Es wird 16 Stunden bei 40° C gerührt. 113 mg (1,31 mmol) Piperazin werden in 15 ml Dioxan gelöst, auf 80° C erhitzt und das Reaktionsgemisch zugetropft. Es wird 2 Stunden gerührt, anschließend in 20 ml Eiswasser getropft. Es wird mit Dichlormethan extrahiert, die organische Phase getrocknet und zur Trockene eingedampft. Der Rückstand wird chromatographisch gereinigt. Entsprechende Fraktion wird zur Trockene eingedampft, dann mit Petrolether/Diethylether verrieben. Ausbeute: 52 mg (= 42% d. Th.)

**Verbindung 29:**
a) 1-(4-Isopropyl-phenyl)-cyclopropylamin: 2,00 g (14 mmol) 4-Isopropylbenzonitril und 4,04 g (13,77 mmol) Titanium(IV)isopropoxid werden in 60 ml Diethylether vorgelegt und auf 70°C gekühlt. 10,10 ml (30,30 mmol) 3 molare Ethylmagnesiumbromid-Lösung werden zugetropft, dann wird 0,1 Stunden nachgerührt. Nach Erwärmen auf Raumtemperatur werden 3,48 ml (27,55 mmol) Bortrifluoridetherat zugetropft und 1 Stunde nachgerührt. Anschließend wird das Reaktionsgemisch mit 25 ml 1 N Salzsäure versetzt, danach werden 32 ml 4 N Natronlauge zugegeben. Der dabei ausfallende Niederschlag wird abgesaugt und verworfen. Das Filtrat wird mit Wasser extrahiert, die organischen Phasen vereinigt, getrocknet und zur Trockene eingedampft. Der Rückstand wird in Dichlormethan aufgenommen, sauer extrahiert und neutralisiert. Die organische Phase wird getrocknet und zur Trockene eingedampft. Der Rückstand wird chromatographisch gereinigt. Ausbeute: 425 mg (=18% d. Th.)
b) (6-Chloro-2-piperazin-1-yl-4-pyrrolidin-1-yl-pteridin-7-yl)-[1-(4-isopropyl-phenyl)-cyclopropyl]-amin *(Beispiel 29)*: 80 mg (0,263 mmol) 2,6,7-Trichlor-4-pyrrolidin-1-yl-pteridin werden in 5 ml Dioxan suspendiert, 51,0 mg (0,290 mmol) 1-(4-Isopropyl-phenyl)-cyclopropylamin und 0,064 ml (0,368 mmol) Diisopropylethylamin werden zugegeben. Es wird 72 Stunden bei 40°C gerührt. 113 mg (1,31 mmol) Piperazin werden in 15 ml Dioxan gelöst, auf 80°C erhitzt und das Reaktionsgemisch zugetropft. Es wird 2 Stunden gerührt, anschließend in Eiswasser getropft. Es wird mit Dichlormethan extrahiert, die organische Phase getrocknet und zur Trockene eingedampft. Der Rückstand wird chromatographisch gereinigt. Entsprechende Fraktion wird zur Trockene eingedampft, dann mit Petrolether/Diethylether verrieben. Ausbeute: 77,50 mg (= 60% d. Th.)

**Verbindung 31:**
a) (S)-Amino-(4-fluorphenyl)-essigsäuremethylester: 500 mg (2,96 mmol) (*S*)- 4-Fluorphenylglycin werden in 10 ml Methanol suspendiert, unter Eiskühlung werden vorsichtig 0,43 ml (5,91 mmol) Thionylchlorid zugetropft. Es wird 16 Stunden bei Raumtemperatur gerührt, dann zur Trockene eingedampft. Ausbeute: 700 mg
b) (4-Fluorphenyl)-(2,2,2-trifluoracetylamino)-essigsäuremethylester: 700 mg (3,19 mmol) (S)-Amino-(4-fluorphenyl)-essigsäuremethylester werden in 5 ml Tetrahydrofuran suspendiert, es werden 0,53 ml (40 mmol) Triethylamin zugegeben. Es wird auf -70° C abgekühlt, dann werden 0,54 ml (40 mmol) Trifluoressigsäureanhydrid zugetropft. Nach Wegnahme der Kühlung wird das Reaktionsgemisch 16 Stunden bei Raumtemperatur gerührt. Anschließend wird mit Wasser und mit Kaliumhydrogencarbonat versetzt, dann mit Ethylacetat extrahiert. Die vereinigten organischen Phasen werden gewaschen, getrocknet und zur Trockene eingedampft. Ausbeute: 680 mg (=76% d. Th.)
c) (S)-2,2,2-Trifluor-N-[1-(4-fluorphenyl)-2-hydroxy-2-methyl-propyl]-acetamid: 680 mg (2,44 mmol) (S)-(4-Fluorphenyl)-(2,2,2-trifluoracetylamino)-essigsäuremethylester werden in 20 ml Tetrahydrofuran vorgelegt, dann wurden 4,06 ml (12,18 mmol) Methylmagnesiumjodid-Lösung langsam zugetropft. Die Temperatur sollte 20°C nicht übersteigen. Das Reaktionsgemisch mit unlöslichem Niederschlag wird 16 Stunden bei Raumtemperatur gerührt, dann auf Eiswasser gegossen . Es erfolgt Zugabe von Ammoniumchlorid bis der Niederschlag gelöst ist. Die wässrige Phase wird mit Diethylether extrahiert, die vereinigten organischen Phasen getrocknet und zur Trockene eingedampft. Ausbeute: 570 mg (= 84% d. Th)
d) (S)-1-Amino-1-(4-fluorphenyl)-2-methyl-propan-2-ol: 570 mg (2,04 mmol) (S)-2,2,2-Trifluor-N-[1-(4-fluorphenyl)-2-hydroxy-2-methyl-propylj-acetamid und 221 mg (40 mmol) Kaliumhydroxid werden in 7 ml Methanol vorgelegt und 16 Stunden bei 60°C gerührt. Anschließend wird das Reaktionsgemisch mit Wasser versetzt und mit Dichlormethan extrahiert. Die organischen Phasen werden vereinigt, getrocknet und zur Trockene eingedampft. Ausbeute: 300 mg (= 80% d. Th.)
e) 1-(6-Chloro-2-piperazin-1-yl-4-pyrrolidin-1-yl-pteridin-7-ylamino)-(*S*)-1-(4-fluorphenyl)-2-methyl-propan-2-ol *(Beispiel 31)*: 100 mg (0,279 mmol) (S)-2,6,7-Trichlor-4-pyrrolidin-1-yl-pteridin werden in 5 ml Dioxan gelöst, 0,053 ml (0,307 mmol) Diisopropylethylamin und 66,20 mg (0,360 mmol) 1-Amino-1-(4-fluorphenyl)-2-methyl-propan-2-ol werden zugegeben, dann wird auf 40°C erhitzt. Das Reaktionsgemisch wird 16 Stunden gerührt. 120 mg (1,40 mmol) Piperazin werden in 5 ml Dioxan gelöst, auf 80° C erhitzt und das Reaktionsgemisch zugetropft. Es wird 16 Stunden gerührt, anschließend wird die Reaktionslösung im Vakuum konzentriert und in Eiswasser getropft. Der ausfallende Niederschlag wird abgesaugt und chromatographisch gereinigt. Entsprechende Fraktionen werden vereinigt, zur Trockene eingedampft. Der Rückstand wird in Dioxan aufgenommen und gefriergetrocknet. Ausbeute: 75 mg (= 54% d. Th.)

**Verbindung 34:**
a) Benzaldehyd tosyl hydrazon: 5,00 g (26,85 mmol) p-Toluolsulfonylhydrazid werden in 10 ml Methanol vorgelegt, 2,37 ml (23,30 mmol) Benzaldehyd langsam zugetropft. Es fällt ein Niederschlag aus. Dieser wird abgesaugt und mit Methanol gewaschen. Anschließend wird der Niederschlag aus Methanol umkristallisiert. Ausbeute: 3,14 g (43% d. Th.)
b) Racemic-cis-2-(2-phenyl-cyclopropyl)-isoindol-1,3-dion: 2,00 g (7,29 mmol) Benzaldehyd tosyl hydrazon werden in 40 ml Tetrahydrofuran vorgelegt und auf -70° C gekühlt. 7,29 ml (7,29 mmmol) LiHMDS Lithiumbis(trimethylsilyl)amid (1 M Lösung in Tetrahydrofuran) werden zugegeben, dann 0,25 Stunden bei -78° C gerührt. Das Reaktionsgemisch wird langsam auf Raumtemperatur erwärmt, dann eingedampft. Der Rückstand wird in 50 ml Dioxan gelöst, mit 0,166 g (1 mmol) Benzyltriethylammoniumchlorid chlorid und 0,032 g Rhodiumacetat-dimer versetzt, 6,31 g (36,45 mmol) 2-Vinyl-isoindole-1,3-dion zugegeben. Das Reaktionsgemisch wird 80 Stunden gerührt, anschließend mit Wasser und Dichlormethan extrahiert. Die organische Phase wird getrocknet und zur Trockene eingedampft. Der Rückstand wird chromatographisch gereinigt. Ausbeute: 0,650 g (34% d. Th.)
c) Racemic-cis-2-phenyl-cyclopropylamin: 0,460 g (1,75 mmol) 2-(2-Phenyl-cyclopropyl)-isoindol-1,3-dion (chiral) werden in 6 ml Ethanol vorgelegt, 0,115 g (1,83 mmol) Hydrazinhydrat in 12 ml Ethanol gelöst zugegeben. Das Reaktionsgemisch wird 15 Stunden bei 40° C gerührt. Anschließend werden 0,58 ml 1 N Salzsäure zugegeben, dann 3 Stunden gerührt. Nach Abkühlen wird ausgefallener Niederschlag abgesaugt, mit Ethanol gewaschen. Das Filtrat wird eingedampft, der Rückstand in 12 ml 1 N Salzsäure aufgenommen und mit Diethylether extrahiert. Die wässrige Phase wird basisch gestellt, mit Dichlormethan extrahiert. Die organische Phase wird getrocknet und zur Trockene eingedampft. Ausbeute: 0,090 g (39% d. Th.)

**Verbindung 35:**
a) Racemic-cis-essigsäure-2-phenyl-cyclopropyl ester: 10,00 g (36,45 mmol) Benzaldehyd tosyl hydrazone werden in 200 ml Tetrahydrofuran vorgelegt und auf -70°C gekühlt. 36,45 ml (36,45 mmol) LiHMDS Lithiumbis(trimethylsilyl)amid (1 molare Lösung in Tetrahydrofuran) werden zugegeben, dann 0,25 Stunden bei -78° C gerührt. Das Reaktionsgemisch wird langsam auf Raumtemperatur erwärmt, dann im Vakuum eingedampft. Der Rückstand wird in 250 ml Dioxan gelöst, mit 0,830 g (4 mmol) Benzyltriethylammoniumchlorid chlorid und 0,161 g Rhodiumacetat-dimer versetzt, 33,59 ml (36,45 mmol) Essigsäurevinylester zugegeben. Das Reaktionsgemisch wird 70 Stunden gerührt, anschließend mit Wasser und Dichlormethan extrahiert. Die organische Phase wird mit konz. Natriumchloridlösung gewaschen, getrocknet und zur Trockene eingedampft. Der Rückstand wird chromatographisch gereinigt. Ausbeute: 0,800 g (11% d. Th.)
b) Racemic-cis-2-phenyl-cyclopropanol: 0,280 g (1,59 mmol) Essigsäure-2-phenyl-cyclopropyl ester (chiral) werden unter Argon in 1,50 ml Diethylether gelöst, 2,00 ml (3,20 mmol) Methyllithium in 2 ml Diethylether gelöst innerhalb 0,25 Stunden zugetropft. Das Reaktionsgemisch wird 0,5 Stunden bei Raumtemperatur gerührt, anschließend in 6 ml konz. Borsäure gegeben. Es wird mit Wasser verdünnt und extrahiert. Die organische Phase wird mit gesättigter Natriumchloridlösung gewaschen, getrocknet und zur Trockene eingedampft. Ausbeute: 0,200 g (94% d. Th.)

Im Folgenden sind beispielhaft Verbindungen zusammengefasst, die analog einem der oben gezeigten Synthesewege dargestellt werden können.

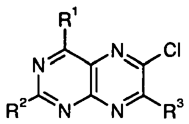

| # | **R¹** | **R²** | **R³** | **M+H** |
|---|---|---|---|---|
| 1. | 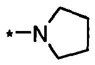 | 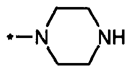 | 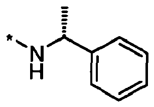 | 439 / 441 |
| 2. | 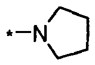 | 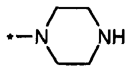 | 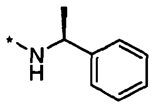 | 439 / 44 |
| 3. | 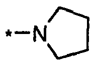 | 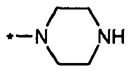 | 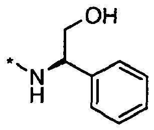 | 455 / 457 |
| 4. | 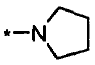 | 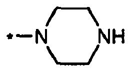 | 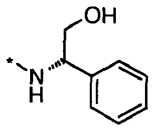 | 455 / 457 |
| 5. | 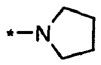 | 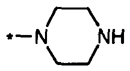 | 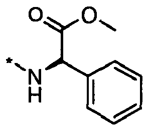 | 483 / 485 |
| 6. | 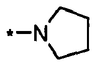 | 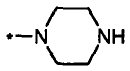 | 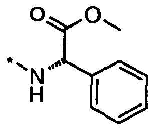 | 483 / 485 |
| 7. | 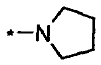 | 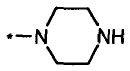 | 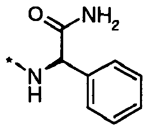 | 468 / 470 |
| 8. | 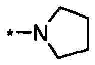 | 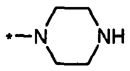 | 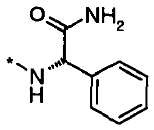 | 468 / 470 |
| 9. | 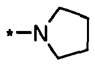 | 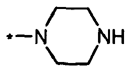 | 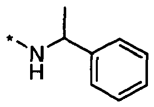 | 439 / 441 |
| 10. | 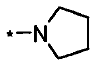 | 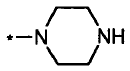 | 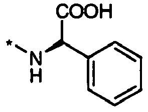 | 469 / 471 |
| 11. | 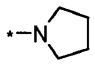 | 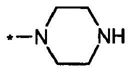 | 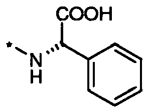 | 469 / 471 |
| 12. | 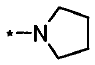 | 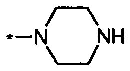 | 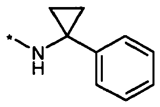 | 451 / 453 |
| 13. | 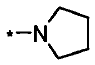 | 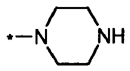 | 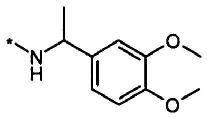 | 499 / 450 |
| 14. | 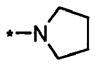 | 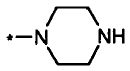 | 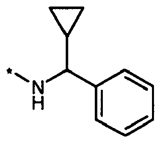 | 465 / 467 |
| 15. | 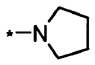 | 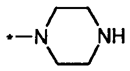 | 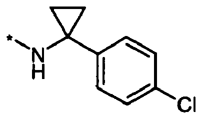 | 485 / 487 / 489 |
| 16. | 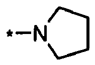 | 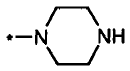 | 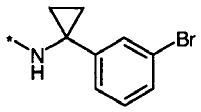 | 529 / 531 / 533 |
| 17. | 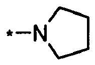 | 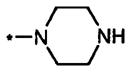 | 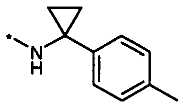 | 465 / 467 |
| 18. | 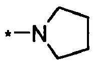 | 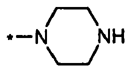 | 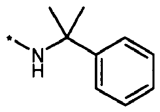 | 453 / 455 |
| 19. | 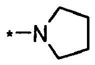 | 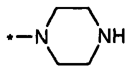 | 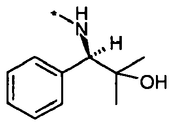 | 483 / 485 |
| 20. | 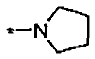 | 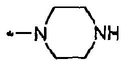 | 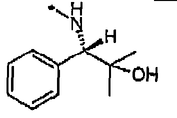 | 483 / 485 |
| 21. | 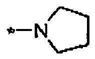 | 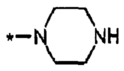 | 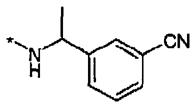 | 464 / 466 |
| 22. | 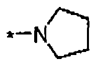 | 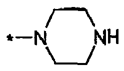 | 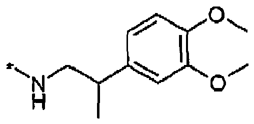 | 513 / 515 |
| 23. | 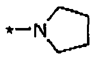 | 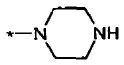 | 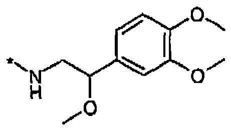 | 529 / 531 |
| 24. | 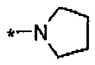 | 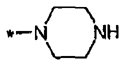 | 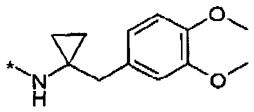 | 525 / 527 |
| 25. | 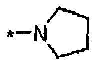 | 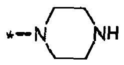 | 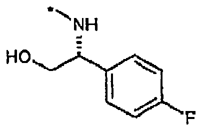 | 473 / 475 |
| 26. | 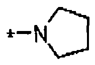 | 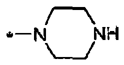 | 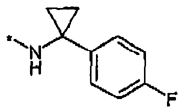 | 469 / 471 |
| 27. | 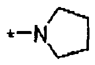 | 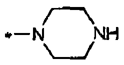 | 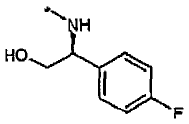 | 473 / 475 |
| 28. | 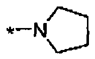 | 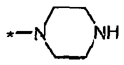 | 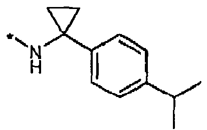 | 4931495 |
| 29. | 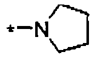 | 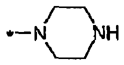 | 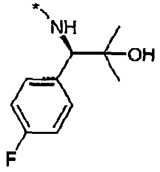 | 501 / 503 |
| 30. | 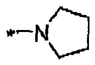 | 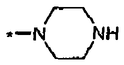 | 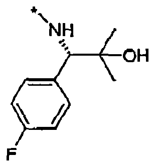 | 501 / 503 |
| 31. | 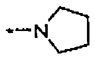 | 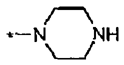 | 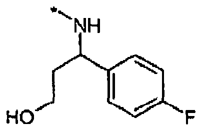 | 687 / 689 |
| 32. | 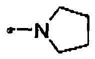 | 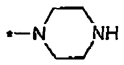 | 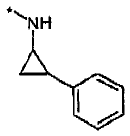 | 451 / 453 |
| 33. | 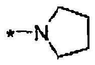 | 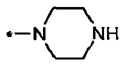 | 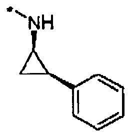 | 451 / 453 |
| 34. | 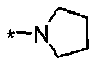 | 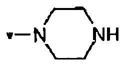 | 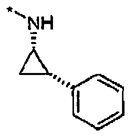 | 451 / 453 |
| 35. | 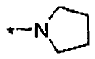 | 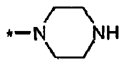 | 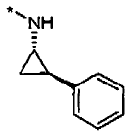 | 451 / 453 |
| 36. | 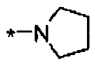 | 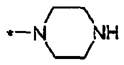 | 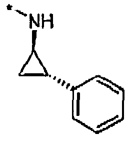 | 451 / 453 |
| 37. | 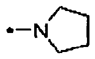 | 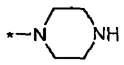 | 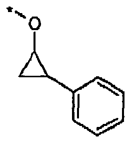 | 451 / 453 |
| 38. | 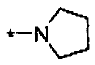 | 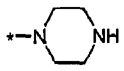 | 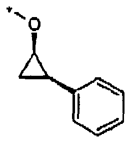 | 451 / 453 |
| 39. | 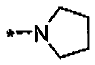 | 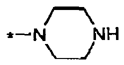 | 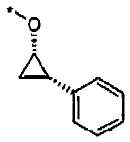 | 451 / 453 |
| 40. | 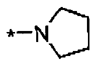 | 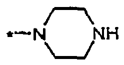 | 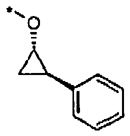 | 451 / 453 |
| 41. | 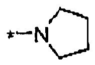 | 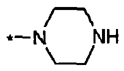 | 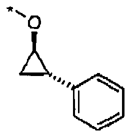 | 451 / 453 |

### Indikationsgebiete

Wie gefunden wurde, zeichnen sich die Verbindungen der Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alphal-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung von Verbindungen der Formel **1** zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der Verbindungen der Formel **1** zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel **1** zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein herausragender Aspekt der vorliegenden Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmengen, ohne Emesis oder Nausea auszulösen, in jedem Stadium des Krankheitsverlaufs.

### KOMBINATIONEN

Die Verbindungen der Formel **1** können allein oder in Kombination mit anderen erfindungsgemäßen Wirkstoffen der Formel **1** zur Anwendung gelangen. Gegebenenfalls können die Verbindungen der Formel **1** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weitere PDE4-Inhibitoren, LTD4-Antagonisten, EGFR-Hemmern, Dopamin-Agonisten, H1-Antihistaminika, PAF-Antagonisten und PI3-Kinase Inhibitoren oder zwei- oder dreifach Kombinationen davon, wie beispielsweise Kombinationen von
- Betamimetika mit Corticosteroiden, PDE4-Inhibitoren, EGFR-Hemmern oder LTD4-Antagonisten,
- Anticholinergika mit Betamimetika, Corticosteroiden, PDE4-Inhibitoren, EGFR-Hemmern oder LTD4-Antagonisten,
- Corticosteroiden mit PDE4-Inhibitoren, EGFR-Hemmern oder LTD4-Antagonisten
- PDE4-Inhibitoren mit EGFR-Hemmern oder LTD4-Antagonisten
- EGFR-Hemmern mit LTD4-Antagonisten.

Auch die Kombinationen dreier Wirkstoffe je einer der o.g. Verbindungsklassen ist Bestandteil der Erfindung.

### DARREICHUNGSFORMEN

Einen weiteren Aspekt der Erfindung bilden Medikamente zur Behandlung von Atemwegserkrankungen, die eines oder mehrere der oben genannten Pteridine der Formel **1** enthalten, das in Kombination mit einem oder mehreren zusätzliche Wirkstoffen ausgewählt aus der Gruppe der Betamimetika, Anticholinergika, Corticosteroide, Pl3-Kinase Inhibitoren, LTD4-Antagonisten, EGFR-Hemmer, Dopamin-Agonisten, H1-Antihistaminika oder PAF-Antagonisten, bevorzugt Betamimetika, Anticholinergika oder Corticosteroide zusammen oder nacheinander, zur simultanen, sequentiellen oder separaten Verabreichung, eingesetzt werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel 1 gemäß der obigen bevorzugten Ausführungsformen.

Besonders bevorzugt ist es, wenn die Verbindungen der Formel 1 oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss-oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmack-Aufbesserern oder Farbstoffen versetzt werden.

Ebenfalls bevorzugt ist es, wenn die Verbindungen der Formel 1 inhalativ verabreicht werden, besonders bevorzugt ist es, wenn die Verabreichung ein oder zweimal täglich erfolgt. Hierzu müssen die Verbindungen der Formel 1 in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel 1 im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können 1 im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1,1,1,2-Tetrafluorethan), TG227 (1,1,1,2,3,3,3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel 1 erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure.

Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, ein Pteridin und ein oder mehrere Kombinationspartner ausgewählt aus der oben beschriebenen Gruppe, bereit gestellt.

## Patentansprüche

1. Verbindungen der Formel **1**, 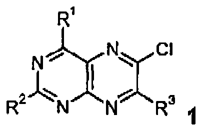 worin
R¹ ein gesättigter oder ungesättigter, fünf-, sechs- oder siebengliedriger, heterocyclischer Ring, der ein Stickstoffatom und ein weiteres Atom ausgewählt aus der Gruppe bestehend aus Stickstoff, Schwefel und Sauerstoff enthalten kann;
R² ein fünf- , sechs- oder siebengliedriger, heterocyclischer Ring der ein Stickstoffatom und ein weiteres Atom ausgewählt aus der Gruppe bestehend aus Stickstoff, Schwefel und Sauerstoff enthalten kann;
R³ eine Gruppe der Formel **1a**, 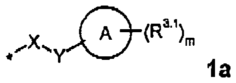 worin
A Aryl, wobei das Aryl für ein aromatisches Ringsystem mit 6 oder 10 Kohlenstoffafiomen steht,
X NR^{3.2},
Y C₁₋₄-Alkylen, substituiert mit einem oder mehreren R^{3.3}
m 0, 1, 2;
R^{3.1} jeweils unabhängig voneinander C₁₋₆₋Alkyl, COOR^{3.1-1}, CONR^{3-1.1}R^{3.1.2}, CN, NR^{3.1.1}R^{3.1.2}, NHCOR^{3.1.1}, OR^{3.1.1}, O-C₁₋₆-Haloalkyl, SO₂R^{3.1.1}, SO₂NH₂, Halogen, C₁₋₆-Haloalkyl, C₁₋₆-alkyl-CONH₂, O-C₁₋₆-alkyl-NH₂, O-C₃₋₈-Cycloalkyl, O-C₁₋₄-Alkylen-C₃₋₆-cycloalkyl oder Aryl, wobei das Aryl für ein aromatisches Ringsystem mit 6 oder 10 Kohlenstoffatomen steht; R^{3.1.1} H, C₁₋₆-Alkyl; R^{3.1.1} H, C₁₋₆-Alkyl; oder
R^{3.1} bildet gemeinsam mit zwei Atomen von A einen 5 oder 6-gliedrigen carbocyclischen Ring oder einen 5 oder 6-gliedrigen heterocyclischen Ring, der ein oder mehrere Sauerstoff- oder Stickstoffatome enthalten kann;
R^{3.2} H, C₁₋₆-Alkyl;
R^{3.3} jeweils unabhängig voneinander C₁₋₆-Alkyl, C₁₋₆-Alkyl-OH, C₃₋₆-Cycloalkyl, C₃₋₆-Cycloalkyl-OH, O-C₁₋₆-Alkyl, COOH, COO-C₁₋₆-Alkyl, CONH₂;
R^{3.3} bildet gemeinsam mit einem oder zwei Kohlenstoffatomen von Y einen carbocyclischen Ring mit 3, 4, 5 oder 6 Kohlenstoffatomen, wobei jedes zuvor genannte Aryl-Gruppe und auch jede zuvor genannte C₃₋₆-Cycloalkyl-Gruppe optional mit einem oder mehreren Resten ausgewählt aus der Gruppe bestehend aus Methyl, Ethyl, iso-Propyl, tert-Butyl; Hydroxy, Fluor, Chlor, Brom und Jod substituiert sein kann, und wobei jeder zuvor genannte heterocyclische Ring optional mit einer Ketogruppe substituiert sein kann,
bedeuten, sowie pharmakologisch verträgliche Salze, Diastereomere, Enantiomere, Racemate, Hydrate oder Solvate davon.

2. Verbindungen der Formel 1, nach Anspruch 1 worin
R¹ ein gesättigter oder ungesättigter, fünf- oder sechsgliedriger, heterocyclischer Ring, der ein Stickstoffatom und ein weiteres Atom ausgewählt aus der Gruppe bestehend aus Stickstoff und Schwefel enthalten kann;
R² ein fünf- oder sechsgliedriger, heterocyclischer Ring, der ein oder zwei Stickstoffatome enthalten kann;
bedeuten, sowie pharmakologisch verträgliche Salz, Diastereomere, Enantiomere, Racemate, Hydrate oder Solvate davon.

3. Verbindungen der Formel **1**, nach Anspruch 1 oder 2 worin
R¹ ein gesättigter oder ungesättigter, fünf- oder sechsgliedriger, heterocyclischer Ring, der ein Stickstoffatom und gegebenenfalls ein weiteres Schwefelatom enthält;
R² ein sechsgliedriger, heterocyclischer Ring, der zwei Stickstoffatome enthält;
bedeuten, sowie pharmakologisch verträgliche Salz, Diastereomere, Enantiomere, Racemate, Hydrate oder Solvate davon.

4. Verbindungen der Formel 1, nach einem der Ansprüche 1 bis 3, worin
R³ ist eine Gruppe der allgemeinen Formel 1a, worin
A Phenyl;
X NR^{3.2},
Y C₁₋₄-Alkylen, substituiert mit einem oder mehreren R^{3.3}
m 0, 1 oder 2;
R^{3.1} jeweils unabhängig voneinander C₁₋₄-Alkyl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₄-Haloalkyl, SO₂R^{3.1.1}, SO₂NH₂, Halogen oder Aryl, wobei das Aryl für ein aromatisches Ringsystem mit 6 oder 10 Kohlenstoffatomen steht;
R^{3.1.1} H, C₁₋₆-Alkyl;
R^{3.1.2} H, C₁₋₆-Alkyl;
R^{3.2} H, C₁₋₆-Alkyl;
R^{3.3} jeweils unabhängig voneinander C₁₋₆-Alkyl, C₁₋₆-Alkyl-OH, C₃₋₆-Cycloalkyl, O-C₁₋₆-Alkyl, COOH, COO-C₁₋₆-Alkyl, CONH₂;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

5. Verbindungen der Formel **1**, nach einem der Ansprüche 1 bis 4, worin
R³ ist eine Gruppe der allgemeinen Formel 1a, worin
A Phenyl;
X NR^{3.2};
Y C₁₋₂-Alkylen, substituiert mit einem oder mehreren R^{3.3}
m 0, 1 oder 2;
R^{3.1} jeweils unabhängig voneinander C₁₋₄-Alkyl, , COOH, COO-C₁₋₄-Alkyl, CONH₂, CN, NH₂, NHCO-C₁₋₄-Alkyl, OH, O-C₁₋₄-Alkyl, O-C₁₋₄-Haloalkyl, SO₂-C₁₋₄-Alkyl, SO₂NH₂, Halogen oder Aryl, wobei das Aryl für ein aromatisches Ringsystem aus 6 oder 10 Kohlenstoffatomen steht;
R^{3.2} H, C₁₋₄-Alkyl;
R^{3.3} jeweils unabhängig voneinander C₁₋₄-Alkyl, C₁₋₄-Alkyl-OH, C₃₋₆-Cycloalkyl, O-C₁₋₄-Alkyl, COOH, COO-C₁₋₄-Alkyl, CONH₂;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

6. Verbindungen der Formel **1**, nach einem der Ansprüche 1 bis 5, worin
R³ ist eine Gruppe der allgemeinen Formel 1a, worin
A Phenyl;
X NR^{3.2};
Y C₁₋₂-Alkylen, substituiert mit einem oder mehreren R^{3.3}
m 0, 1 oder 2;
R^{3.1} jeweils unabhängig voneinander Methyl, Ethyl, Propyl, Phenyl, COOH, COOMe, CONH₂, CN, NH₂, NHCOMe, OH, OMe, OEt, OCF₃, OCHF₂, SO₂Me, SO₂NH₂, F, Cl, Br;
R^{3.2} H, C₁₋₄-Alkyl;
R^{3.3} jeweils unabhängig voneinander Methyl, Ethyl, Propyl, Butyl, CH₂OH, CH₂CH₂OH, C(CH₂)₂OH, Cyclopropyl, COOH, COOMe, COOEt, COOPr CONH₂, OMe, OEt, OPr;
R^{3.3} bildet gemeinsam mit einem oder zwei Kohlenstoffatomen von Y einen carbocyclischen Ring mit 3, 5 oder 6 Kohlenstoffatomen
bedeutet, sowie pharmakologisch verträgliche Salz, Diastereomere, Enantiomere, Racemate, Hydrate oder Solvate davon.

7. Verbindungen der Formel **1**, nach einem der Ansprüche 1 bis 6, worin
R³ ist eine Gruppe der allgemeinen Formel **1a**, worin
A Phenyl;
X NR^{3.2};
Y C₁₋₂-Alkylen, substituiert mit einem oder mehreren R^{3.3}
m 0, 1 oder 2;
R^{3.1} jeweils unabhängig voneinander Methyl, iso-Propyl, OMe, F, Cl, Br, CN,
R^{3.2} H;
R^{3.3} jeweils unabhängig voneinander Methyl, Cyclopropyl, CH₂OH, CH₂CH₂OH, C(CHO₂)₂OH, COOH, COOMe, CONH₂, OMe,
R^{3.3} bildet gemeinsam mit einem oder zwei Kohlenstoffatomen von Y einen carbocyclischen Ring mit 3 Kohlenstoffatomen
bedeutet, sowie pharmakologisch verträgliche Salz, Diastereomere, Enantiomere, Racemate, Hydrate oder Solvate davon.

8. Verbindungen nach einem der Ansprüche 1 bis 7 als Arzneimittel.

9. Verwendung von Verbindungen nach einem der Ansprüche 1 bis 7, zur Herstellung eines Medikaments zur Behandlung von Krankheiten, die sich durch Inhibition des PDE4-Enzyms behandeln lassen.

10. Verwendung von Verbindungen nach einem der Ansprüche 1 bis 7 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

11. Verwendung von Verbindungen nach einem der Ansprüche 1 bis 7 zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einher gehen.

12. Verwendung von Verbindungen nach einem der Ansprüche 1 bis 7 zur Herstellung eines Medikaments zur Behandlung von entzündlichen Erkrankungen des Gastrointestinaltraktes.

13. Verwendung von Verbindungen nach einem der Ansprüche 1 bis 7 zur Herstellung eines Medikament zur Behandlung von entzündlichen und obstruktiven Erkrankungen wie COPD, chronischer Sinusitis, Asthma, Morbus Crohn, Colitis ulcerosa.

14. Verwendung von Verbindungen nach einem der Ansprüche 1 bis 7 zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

15. Verwendung von Verbindungen nach einem der Ansprüche 1 bis 7 zur Herstellung eines Medikaments zur Behandlung von Krebserkrankungen wie akuten und chronischen Leukämien, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastorn.

## Claims

1. Compounds of formula 1, 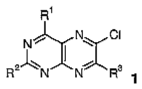 wherein
R' denotes a saturated or unsaturated, five-, six- or seven-membered heterocyclic ring which may contain a nitrogen atom and another atom selected from among nitrogen, sulphur and oxygen;
R² denotes a five-, six- or seven-membered heterocyclic ring which may contain a nitrogen atom and another atom selected from among nitrogen, sulphur and oxygen;
R³ denotes a group of formula **1a**, 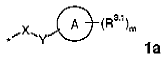 wherein
A denotes aryl, wherein the aryl denotes an aromatic ring system with 6 or 10 carbon atoms,
X denotes NR^{3.2},
Y denotes C₁₋₄-alkylene, substituted by one or more R^{3.3}
m denotes 0, 1, 2;
R^{3.1} each independently of one another denote C₁₋₆-alkyl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₆-haloalkyl, SO₂R^{3.1.1}, SO₂NH₂, halogen, C₁₋₆-haloalkyl, C₁₋₆-alkyl-CONH₂, O-C₁₋₆-alkyl-NH₂, O-C₃₋₆-cycloalkyl, O-C₁₋₄-alkylene-C₃₋₆-cycloalkyl or aryl, wherein the aryl denotes an aromatic ring system with 6 or 10 carbon atoms;
R^{3.1.1} denotes H, C₁₋₆-alkyl;
R^{3.1.1} denotes H, C₁₋₆-alkyl; or
R^{3.1} together with two atoms of A forms a 5- or 6-membered carbocyclic ring or a 5- or 6-membered heterocyclic ring which may contain one or more oxygen or nitrogen atoms;
R^{3.2} denotes H, C₁₋₆-alkyl;
R^{3.3} each independently of one another denote C₁₋₆-alkyl, C₁₋₆-alkyl-OH, C₁₋₆-cycloalkyl, C₃₋₆-cycloalkyl-OH, O-C₁₋₆-alkyl, COOH, COO-C₁₋₆-alkyl, CONH₂;
R^{3.3} together with one or two carbon atoms of Y forms a carbocyclic ring with 3, 4, 5 or 6 carbon atoms
wherein each above-mentioned aryl group and also each above-mentioned C₃₋₆-cycloalkyl group may optionally be substituted by one or more groups selected from among methyl, ethyl, iso-propyl, tert-butyl; hydroxyl, fluorine, chlorine, bromine and iodine,
and wherein each above-mentioned heterocyclic ring may optionally be substituted by a keto group,
and pharmacologically acceptable salts, diastereomers, enantiomers, racemates, hydrates or solvates thereof.

2. Compounds of formula 1 according to claim 1, wherein
R¹ denotes a saturated or unsaturated, five- or six-membered heterocyclic ring which may contain a nitrogen atom and another atom selected from among nitrogen and sulphur;
R² denotes a five- or six-membered heterocyclic ring which may contain one or two nitrogen atoms;
and pharmacologically acceptable salts, diastereomers, enantiomers, racemates, hydrates or solvates thereof.

3. Compounds of formula **1** according to claim 1 or 2, wherein
R¹ denotes a saturated or unsaturated, five- or six-membered heterocyclic ring which may contain a nitrogen atom and optionally contains a further sulphur atom;
R² contains a six-membered heterocyclic ring which contains two nitrogen atoms;
and pharmacologically acceptable salt, diastereomers, enantiomers, racemates, hydrates or solvates thereof.

4. Compounds of formula 1 according to one of claims 1 to 3, wherein
R³ is a group of general formula 1a, wherein
A denotes phenyl;
X denotes NR^{3.2},
Y denotes C₁₋₄-alkylene, substituted by one or more R^{3.3}
m denotes 0, 1 or 2;
R³⁻¹ each independently of one another denote C₁₋₄-alkyl, COOR^{3.1.1}, CONR^{3.1.1}R^{3.1.2}, CN, NR^{3.1.1}R^{3.1.2}, NHCOR^{3.1.1}, OR^{3.1.1}, O-C₁₋₄-haloalkyl, SO₂R^{3.1.1}, SO₂NH₂, halogen or aryl, wherein the aryl denotes an aromatic ring system with 6 or 10 carbon atoms;
R^{3.1.1} denotes H, C₁₋₆-alkyl;
R^{3.1.2} denotes H, C₁₋₆-alkyl;
R^{3.2} denotes H, C₁₋₆-alkyl;
R^{3.3} each independently of one another denote C₁₋₆-alkyl, C₁₋₆-alkyl-OH, C₃₋₆-cycloalkyl, O-C₁₋₆-alkyl, COOH, COO-C₁₋₆-alkyl, CONH₂;
R^{3.3}
together with one or two carbon atoms of Y forms a carbocyclic ring with 3, 5 or 6 carbon atoms and pharmacologically acceptable salt, diastereomers, enantiomers, racemates, hydrates or solvates thereof.

5. Compounds of formula **1**, according to one of claims 1 to 4, wherein
R³ is a group of general formula **1a**, wherein
A denotes phenyl;
X denotes NR^{3.2};
Y denotes C₁₋₂-alkylene, substituted by one or more R^{3.3}
m denotes 0, 1 or 2;
R^{3.1} each independently of one another denote C₁₋₄-alkyl, COOH, COO-C₁₋₄-alkyl, CONH₂, CN, NH₂, NHCO-C₁₋₄-alkyl, OH, O-C₁₋₄-alkyl, O-C₁₋₄-haloalkyl, SO₂-C₁₋₄-alkyl, SO₂NH₂, halogen or aryl, wherein the aryl denotes an aromatic ring system with 6 or 10 carbon atoms;
R^{3.2} denotes H, C₁₋₄-alkyl;
R^{3.3} each independently of one another denote C₁₋₄-alkyl, C₁₋₄-alkyl-OH, C₃₋₆-cycloalkyl, O-C₁₋₄-alkyl, COOH, COO-C₁₋₄-alkyl, CONH₂;
R^{3.3} together with one or two carbon atoms of Y forms a carbocyclic ring with 3, 5 or 6 carbon atoms
and pharmacologically acceptable salt, diastereomers, enantiomers, racemates, hydrates or solvates thereof.

6. Compounds of formula **1**, according to one of claims 1 to 5, wherein
R³ is a group of general formula **1a**, wherein
A denotes phenyl;
X denotes NR^{3.2};
Y denotes C₁₋₂-alkylene, substituted by one or more R^{3.3} m denotes 0, 1 or 2;
R^{3.1} each independently of one another denote methyl, ethyl, propyl, phenyl, COOH, COOMe, CONH₂, CN, NH₂, NHCOMe, OH, OMe, OEt, OCF₃, OCHF₂, SO₂Me, SO₂NH₂, F, Cl, Br;
R^{3.2} denotes H, C₁₋₄-alkyl;
R^{3.3} each independently of one another denote methyl, ethyl, propyl, butyl, CH₂OH, CH₂CH₂OH, C(CH₂)₂OH, cyclopropyl, COOH, COOMe, COOEt, COOPr CONH₂, OMe, OEt, OPr;
R^{3.3} together with one or two carbon atoms of Y forms a carbocyclic ring with 3, 5 or 6 carbon atoms
and pharmacologically acceptable salt, diastereomers, enantiomers, racemates, hydrates or solvates thereof.

7. Compounds of formula **1**, according to one of claims 1 to 6, wherein
R³ is a group of general formula **1a**, wherein
A denotes phenyl;
X denotes NR³²;
Y denotes C₁₋₂-alkylene, substituted by one or more R^{3.3} m denotes 0, 1 or **2**;
R^{3.1} each independently of one another denote methyl, *iso*-propyl, OMe, F, Cl, Br, CN,
R^{3.2} denotes H;
R^{3.3} each independently of one another denote methyl, cyclopropyl, CH₂OH, CH₂CH₉OH, C(CH₂)₂OH, COOH, COOMe, CONH₂, OMe,
R^{3.3} together with one or two carbon atoms of Y forms a carbocyclic ring with 3 carbon atoms
and pharmacologically acceptable salt, diastereomers, enantiomers, racemates, hydrates or solvates thereof.

8. Compounds according to one of claims 1 to 7 as pharmaceutical compositions.

9. Use of compounds according to one of claims 1 to 7, for preparing a medicament for the treatment of diseases which can be treated by inhibiting the PDE4 enzyme.

10. Use of compounds according to one of claims 1 to 7 for preparing a medicament for the treatment of respiratory or gastrointestinal complaints or diseases, and also inflammatory diseases of the joints, skin or eyes, cancers, as well as diseases of the peripheral or central nervous system.

11. Use of compounds according to one of claims 1 to 7 for preparing a medicament for the prevention and treatment of respiratory or pulmonary diseases which are accompanied by increased mucus production, inflammation and/or obstructive diseases of the airways.

12. Use of compounds according to one of claims 1 to 7 for preparing a medicament for the treatment of inflammatory diseases of the gastrointestinal tract.

13. Use of compounds according to one of claims 1 to 7 for preparing a medicament for the treatment of inflammatory and obstructive diseases such as COPD, chronic sinusitis, asthma, Crohn's disease, ulcerative colitis.

14. Use of compounds according to one of claims 1 to 7 for preparing a medicament for the prevention and treatment of diseases of the peripheral or central nervous system such as depression, bipolar or manic depression, acute and chronic anxiety states, schizophrenia, Alzheimer's disease, Parkinson's disease, acute and chronic multiple sclerosis or acute and chronic pain as well as injuries to the brain caused by stroke, hypoxia or craniocerebral trauma.

15. Use of compounds according to one of claims 1 to 7 for preparing a medicament for the treatment of cancers such as acute and chronic leukaemias, acute lymphatic and acute myeloid leukaemia, chronic lymphatic and chronic myeloid leukaemia, and bone tumours such as osteosarcoma and all types of glioma such as oligodendroglioma and glioblastoma.

## Revendications

1. Composés de formule 1 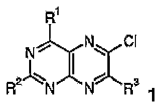 dans laquelle
R¹ est un cycle hétérocyclique saturé ou insaturé, de cinq, six ou sept chaînons, qui peut contenir un atome d'azote et un autre atome choisi dans le groupe comprenant l'azote, le soufre et l'oxygène ;
R² est un cycle hétérocyclique de cinq, six ou sept chaînons qui peut contenir un atome d'azote et un autre atome choisi dans le groupe comprenant l'azote, le soufre et l'oxygène ;
R³ est un groupe de formule 1a 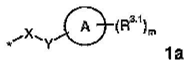 dans laquelle
A est un groupe aryle, le groupe aryle représentant un système cyclique aromatique comportant 6 ou 10 atomes de carbone,
X est NR^{3,2}
Y est un groupe alkylène en C₁ à C₄ substitué par un ou plusieurs R^{3,3}
m est 0, 1, 2 ;
les R^{3,1} désigne respectivement, indépendamment les un des autres, un groupe alkyle en C₁ à C₆, COOR^{3,1,1}, CONR^{3,3,1}R^{3,1,2}, CN, NR^{3,1,2}R^{3,1,2}, NHCOR^{3,1,1}, OR^{3,1,1}, O-halogénoalkyle en C₁ à C₆, SO₂R^{3,1,1}, SO₂NH₂, halogéno, halogénoalkyle en C₁ à C₆, alkyle en C₁ à C₆-CONH₂, O-alkyle en C₁ à C₆-NH₂, O-cycloalkyle en C₃ à C₆, O-alkylène en C₁ à C₄-cycloalkyle en C₃ à C₆ ou aryle, le groupe aryle représentant un système cyclique aromatique de 6 à 10 atomes de carbone ;
R^{3,1,1} est H, un groupe alkyle en C₁ à C₆;
R^{3,1,2} est H, un groupe alkyle en C₁ à C₆ ;ou
R^{3,1} forme conjointement avec deux atomes de A, un groupe carbocyclique de 5 ou 6 chaînons ou un cycle hétérocyclique de 5 ou 6 chaînons qui peut contenir un ou plusieurs atomes d'oxygène ou d'azote ;
R^{3,2} désigne H, un groupe alkyle en C₁ à C₆ ;
les R^{3,3} désignent respectivement indépendamment les uns des autres, un groupe alkyle en C₁ à C₆, alkyle en C₁ à C₆-OH, cycloalkyle en C₃ à C₆, cycloalkyle en C₃ à C₆-OH, O-alkyle en C₁ à C₆, COOH, COO-alkyle en C₁ à C₆, CONH₂ ;
les R^{3,3} forment conjointement avec ou un deux atomes de carbone de Y, un cycle carbocyclique comportant 3, 4, 5 ou 6 atomes de carbone,
respectivement, chacun des groupes aryles précédemment cités et également chacun des groupes cycloalkyle en C₃ à C₆ précédemment cité pouvant être substitué éventuellement par un ou plusieurs radicaux choisis dans le groupe comprenant les groupes méthyle, éthyle, isopropylle, tert-butyle, hydroxy, un atome de fluor, de chlore, de brome et d'iode,
et chaque cycle hétérocyclique précédemment cité pouvant être éventuellement substitué par un groupe céto,
et leurs sels, diastéréomères, énantiomères, racémates, hydrates ou solvates pharmacologiquement acceptables.

2. Composés de formule 1, selon la revendication 1, dans laquelle
R¹ est un cycle hétérocyclique saturé ou insaturé, de cinq ou six chaînons, qui peut contenir un atome d'azote et un autre atome choisi dans le groupe comprenant l'azote et le soufre ;
R² est un cycle hétérocyclique de cinq ou six chaînons qui peut contenir un ou deux atomes d'azote,
et leurs sels, diastéréomères, énantiomères, racémates, hydrates ou solvates pharmacologiquement acceptables.

3. Composés de formule 1, selon la revendication 1 ou 2, dans laquelle
R¹ est un cycle hétérocyclique saturé ou insaturé, de cinq ou six chaînons, qui peut contenir un atome d'azote et éventuellement, un autre atome de soufre ;
R² est un cycle hétérocyclique à six chaînons qui contient deux atomes d'azote,
et leurs sels, diastéréomères, énantiomères, racémates, hydrates ou solvates pharmacologiquement acceptables.

4. Composés de formule 1, selon l'une des revendications 1 à 3, dans laquelle
R³ est un groupe de formule générale 1a, dans laquelle
A est un groupe phényle,
X est NR^{3,2}
Y est un groupe alkylène en C₁ à C₄ substitué par un ou plusieurs R^{3,3}
m est 0, 1, 2 ;
R^{3,1} désigne respectivement, indépendamment les un des autres, un groupe alkyle en C₁ à C₄ , COOR^{3,1,1}, CONR^{3,3,1}R^{3,1,2}, CN, NR^{3,1,2}R^{3,1,2}, NHCOR^{3,1,1}, OR^{3,1,1}, O-halogénoalkyle en C₁ à C₄, SO₂R^{3,1,1}, SO₂NH₂, halogéno ou aryle, le groupe aryle représentant un système cyclique aromatique de 6 à 10 atomes de carbone ;
R^{3,1,1} est H, un groupe alkyle en C₁ à C₆ ;
R^{3,1,2} est H, un groupe alkyle en C₁ à C₆ ;
R^{3,2} désigne H, un groupe alkyle en C₁ à C₆ ;
les R^{3,3} désignent respectivement indépendamment les uns des autres, un groupe alkyle en C₁ à C₆, alkyle en C₁ à C₆-OH, cycloalkyle en C₃ à C₆, O-alkyle en C₁ à C₆, COOH, COO-alkyle en C₁ à C₆, CONH₂ ;
les R^{3,3} forment conjointement avec ou un deux atomes de carbone de Y, un cycle carbocyclique comportant 3, 5 ou 6 atomes de carbone,
et leurs sels, diastéréomères, énantiomères, racémates, hydrates ou solvates pharmacologiquement acceptables.

5. Composés de formule 1, selon l'une des revendications 1 à 4, dans laquelle
R³ est un groupe de formule générale 1a, dans laquelle
A est un groupe phényle,
X est NR^{3,2}
Y est un groupe alkylène en C₁ à C₂ substitué par un ou plusieurs R^{3,3}
m est 0, 1, 2 ;
les R^{3,1} désignent respectivement, indépendamment les un des autres, un groupe alkyle en C₁ à C₄, COOH, COO-alkyle en C₁ à C₄, CONH₂, CN, NH₂, NHCO-alkyle en C₁ à C₄, OH, O-alkyle en C₁ à C₄, O-halogénoalkyle en C₁ à C₄, SO₂-alkyle en C₁ à C₄, SO₂NH₂, halogéno ou aryle, le groupe aryle représentant un système cyclique aromatique de 6 à 10 atomes de carbone ;
R^{3,2} désigne H, un groupe alkyle en C₁ à C₄ ;
les R^{3,3} forment conjointement indépendamment, un groupe alkyle en C₁ à C₄, alkyle en C₁ à C₄-OH, cycloalkyle en C₃ à C₆, O-alkyle en C₁ à C₄, COOH, COO-alkyle en C₁ à C₄, CONH₂ ;
les R^{3,3} forment conjointement avec un ou deux atomes de carbone de Y, un cycle carbocyclique comportant 3, 5 ou 6 atomes de carbone,
et leurs sels, diastéréomères, énantiomères, racémates, hydrates ou solvates pharmacologiquement acceptables.

6. Composés de formule 1 selon l'une des revendications 1 à 5, dans laquelle
R³ est un groupe de formule générale 1a, dans laquelle
A est un groupe phényle,
X est NR^{3,2}
Y est un groupe alkylène en C₁ à C₂ substitué par un ou plusieurs R^{3,3}
m est 0, 1, 2 ;
les R^{3,1} désignent respectivement, indépendamment les uns des autres, un groupe méthyle, éthyle, propyle, phényle, COOH, COOMe, CONH₂, CN, NH₂, NHCOMe, OH, OMe, OEt, OCF₃, OCHF₂, SO₂Me, SO₂NH₂, F, Cl, Br ;
R^{3,2} désigne H, un groupe alkyle en C₁ à C₄ ;
les R^{3,3} forme respectivement, indépendamment les uns des autres, un groupe méthyle, éthyle, propyle, butyle, CH₂OH, CH₂CH₂OH, C(CH₂)₂OH, cyclopropyle, COOH, COOMe, COOEt, COOPr CONH₂, OMe, OEt, OPr ;
les R^{3,3} forment conjointement avec un ou deux atomes de carbone de Y, un cycle carbocyclique comportant 3, 5 ou 6 atomes de carbone,
et leurs sels, diastéréomères, énantiomères, racémates, hydrates ou solvates pharmacologiquement acceptables.

7. Composés de formule 1 selon l'une des revendications 1 à 6, dans laquelle
R³ est un groupe de formule générale 1a, dans laquelle
A est un groupe phényle,
X est NR^{3,2}
Y est un groupe alkylène en C₁ à C₂ substitué par un ou plusieurs R^{3,3}
m est 0, 1, 2 ;
les R^{3,1} désignent respectivement, indépendamment les un des autres, un groupe méthyle, iso-propyle, OMe, F, Cl, Br, CN,
R^{3,2} est H ;
les R^{3,3} forment respectivement, indépendamment les uns des autres, un groupe méthyle, cyclopropyle, CH₂OH, CH₂CH₂OH, C(CH₂)₂OH, COOH, COOMe, CONH₂, OMe ;
les R^{3,3} forment conjointement avec un ou deux atomes de carbone de Y, un cycle carbocyclique comportant 3 atomes de carbone,
et leurs sels, diastéréomères, énantiomères, racémates, hydrates ou solvates pharmacologiquement acceptables.

8. Composés selon l'une des revendications 1 à 7 comme médicament.

9. Utilisation de composés selon l'une des revendications 1 à 7, pour la production d'un médicament destiné au traitement de maladies qui peuvent être traitées par l'inhibition de l'enzyme PDE4.

10. Utilisation de composés selon l'une des revendications 1 à 7, pour la production d'un médicament destiné au traitement des troubles ou des maladies des voies respiratoires ou gastro-intestinales et également des affections des articulations, de la peau ou des yeux, des cancers et des maladies du système nerveux périphérique ou central.

11. Utilisation de composés selon l'une des revendications 1 à 7, pour la production d'un médicament destiné à la prévention et au traitement des maladies des voies respiratoires ou pulmonaires qui s'accompagnent d'une augmentation de la production de mucus, d'inflammations et/ou de maladies obstructives des voies respiratoires.

12. Utilisation de composés selon l'une des revendications 1 à 7, pour la production d'un médicament destiné au traitement des maladies inflammatoires des voies gastro-intestinales.

13. Utilisation de composés selon l'une des revendications 1 à 7, pour la production d'un médicament destiné au traitement de maladies inflammatoires et obstructives telles que la BPCO, la sinusite chronique, l'asthme, la maladie de Crohn, la rectocolite hémorragique.

14. Utilisation de composés selon l'une des revendications 1 à 7, pour la production d'un médicament destiné à la prévention et au traitement de maladies du système nerveux périphérique et central telles que la dépression, la dépression bipolaire ou maniaque, les états d'angoisse aiguë ou chronique, la schizophrénie, la maladie d'Alzheimer, la maladie de Parkinson, la sclérose en plaque aiguë et chronique ou les douleurs aiguës et chroniques et les lésions cérébrales dues à un AVC, une hypoxie ou un traumatisme crânien.

15. Utilisation de composés selon l'une des revendications 1 à 7 pour la production d'un médicament destiné au traitement des cancers tels que les leucémies aiguës et chroniques, les leucémies lymphatiques aiguës et myéloïdes aiguës, les leucémies lymphatiques chroniques et myéloïdes chroniques et les tumeurs osseuses telles que les ostéosarcomes et tous les types de gliomes tels que l'oligodendrogliome et les glioblastomes.
